(19) Europäisches Patentamt European Patent Office Office européen des brevets

(11) **EP 2 163 378 B1**

(12) **EUROPEAN PATENT SPECIFICATION**

(45) Date of publication and mention of the grant of the patent:
**04.01.2017 Bulletin 2017/01**

(51) Int Cl.:
*B65D 85/40* (2006.01)     *B32B 5/06* (2006.01)
*B32B 5/22* (2006.01)     *D04H 1/498* (2012.01)
*A61F 13/551* (2006.01)     *B32B 5/26* (2006.01)
*D04H 1/26* (2012.01)     *D04H 1/28* (2012.01)
*D04H 1/32* (2012.01)     *D04H 1/4258* (2012.01)
*D04H 1/4291* (2012.01)     *D04H 1/435* (2012.01)
*D04H 1/4374* (2012.01)     *D04H 1/46* (2012.01)
*D04H 1/492* (2012.01)     *D21H 27/38* (2006.01)
*D21H 27/42* (2006.01)

(21) Application number: **08777749.6**

(22) Date of filing: **01.07.2008**

(86) International application number:
**PCT/JP2008/061898**

(87) International publication number:
**WO 2009/005057 (08.01.2009 Gazette 2009/02)**

(54) **INDIVIDUAL PACKAGE OF ABSORBENT ARTICLE, WRAPPING SHEET, AND METHOD OF MANUFACTURING WRAPPING SHEET**

EINZELNE PAKETE AUS SAUGFÄHIGEN ARTIKELN, VERPACKUNGSMATERIAL UND VERFAHREN ZUR HERSTELLUNG DES VERPACKUNGSMATERIALS

EMBALLAGES INDIVIDUELS D'ARTICLES ABSORBANTS, FEUILLE D'EMBALLAGE ET PROCÉDÉ DE PRODUCTION DE FEUILLE D'EMBALLAGE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.07.2007 JP 2007174934**

(43) Date of publication of application:
**17.03.2010 Bulletin 2010/11**

(73) Proprietor: **Unicharm Corporation Ehime 799-0111 (JP)**

(72) Inventors:
• **KONISHI, Takayoshi c/o Unicharm Corporation Kagawa 769-1602 (JP)**
• **YAMAKI, Koichi c/o Unicharm Corporation Kagawa 769-1602 (JP)**
• **SUZUKI, Nahomi c/o Unicharm Corporation Kagawa 769-1602 (JP)**

(74) Representative: **Stark, Gordon Drummond Murgitroyd & Company Scotland House 165-169 Scotland Street Glasgow G5 8PL (GB)**

(56) References cited:
**JP-A- 09 228 214**     **JP-A- 10 053 995**
**JP-A- 2000 318 076**     **JP-A- 2002 053 170**
**JP-A- 2003 292 026**     **US-A- 6 010 001**

**Description**

## TECHNICAL FIELD

The present invention relates to an individual package of an absorbent material, a wrapping sheet used for the individual package, and a method of manufacturing the same.

## BACKGROUND ART

Absorbent articles such as sanitary napkins, pantiliners and disposable interlabial pads are each wrapped with an individual wrapping material, which is prepared separately from the absorbent article, for sanitary reasons and for portability reasons. In recent years, absorbent articles with a hydrolytic property (or water degradability) which allows the absorbent articles to be quickly dispersed by a large amount of water have been developed. When such an absorbent article is discarded into a toilet bowl after being used, the absorbent article can be dissolved in the septic tank or the like in a short time without clogging a drainage pipe of the toilet. This leads to reductions of manpower and costs needed for garbage collection and disposal.

Users wear absorbent articles mostly in toilets because of the nature of the absorbent articles. For this reason, it is desired that individual wrapping materials with which the absorbent articles are individually wrapped should be also water-degradable. When individual wrapping materials are not water-degradable, users may sometimes feel inconvenience in disposal of the individual wrapping materials after using the absorbent articles.

With this taken into consideration, for example, paper of a type which is softened through a crepe process for making the paper water-degradable, and which has a basis weight of 20 $g/m^2$ to 40 $g/m^2$, are used as wrapping materials for absorbent articles.

In addition, Japanese Patent Application Publications Nos. Hei 9-228214 and 2001-172850 propose a water-degradable type of fiber sheet with a high wet strength and a good hydrolytic property, which is produced by applying a high-pressure water jetting treatment to a fiber sheet formed by blending predetermined fibers.

Patent Document 1: JP-A 9-228214
Patent Document 2: JP-A 2001-172850

JP 2000 318076 A discloses a wrapping sheet made by laminating fiber layers.

A general type of paper exhibits a poor wet strength although it has good air tightness and hydrolytic properties. However, the general type of paper is easily broken once it becomes wet when touched by a wet hand, or when splashed with water. As a result, a problem with the general type of paper is that it does not function as wrapping material.

In addition, the water-degradable type of fiber sheets with a hydrolytic property, which are disclosed by JP-A 9-228214 and 2001-172850, exhibits flexibility and appropriate degree of wet strength with hydrolytic property. However, the water-degradable type of fiber sheets exhibit a degraded air tightness property in comparison with the general type of paper. This is because the formation of the water-degradable type of fiber sheets is disturbed or fine pores are made in the water-degradable type of fiber sheets, when a high-pressure water jetting treatment is applied to the water-degradable type of fiber sheets. As a result, a problem with the water-degradable type of fiber sheets is that, when used as an individual wrapping material, the water-degradable type of fiber sheets allow foreign matters such as dust to go in beyond the water-degradable type of fiber sheets.

The present invention has been made for the purpose of solving the foregoing problems. An object of the present invention is to provide: an individual package for an absorbent article, the individual package exhibiting a hydrolytic property which allows the individual package to be easily dispersed by water flow; a wrapping material and a wrapping sheet which both exhibits a hydrolytic property; and a method of manufacturing the same.

## DISCLOSURE OF THE INVENTION

The inventors found that a wrapping sheet which reconciles a high hermetic property with a hydrolytic property can be provided in the form of a fiber sheet obtained by laminating two or more layers including a fiber layer with an enhanced hermetic property and a fiber layer with an enhanced soft tactile impression, strength and the like. Thus, the inventors have completed the present invention. Specifically, the present invention provides the following things.

(1) A wrapping sheet for an absorbent article including at least an absorbent exhibiting a fluid-retention property, including: a first fiber layer formed by application of a high-pressure water jetting treatment, the first fiber layer including pulp fiber and chemical fiber, a pulp fiber content of the first fiber layer being 30% by mass to 70% by mass, a chemical fiber content of the first fiber layer being 70% by mass and 30% by mass, an average fiber length of the chemical fiber being not more than 20 mm; and a second fiber layer arranged by laminating the second fiber layer to the first fiber layer, the second fiber layer including pulp fiber, the pulp fiber content of the second fiber layer being 70% by mass to 100% by mass.

(2) The wrapping sheet as recited in (1), in which the second fiber layer includes chemical fiber, the second fiber layer includes chemical fiber hot more than 30% by mass with an average fiber length of 20 mm or less and with a fiber diameter smaller than that of the pulp fiber.

(3) The wrapping sheet as recited in any one of (1) and (2), further including a third fiber layer formed by application of a high-pressure water jetting treatment and arranged by laminating the third fiber layer to one side of the second fiber layer with the first fiber layer laminated to the other side of the second fiber layer, the third fiber layer including pulp fiber and chemical fiber, a pulp fiber content of the third fiber layer being 30% by mass to 70% by mass, a chemical fiber content of the third fiber layer being 70% by mass to 30% by mass, an average fiber length of the chemical fiber being not more than 20 mm.

(4) The wrapping sheet as recited in any one of (1) to (3), in which, when a 10 cmx 10cm sample piece of wrapping sheet placed in 800 cc of distilled water is shaken at a shaking speed of 240 rpm for 30 minutes, the sample piece is so dispersed in the distilled water that the largest remaining piece of wrapping sheet is 50 cm$^2$ or less in size.

(5) The wrapping sheet as recited in any one of (1) to (4), in which: the basis weight of the first fiber layer is 10 g/m$^2$ to 30 g/m$^2$; the basis weight of the second fiber layer is 15 g/m$^2$ to 30 g/m$^2$; and the basis weight of the wrapping sheet is 25 g/m$^2$ to 60 g/m$^2$.

(6) The wrapping sheet as recited in any one of (3) and (4), in which: the basis weight of the first fiber layer is 10 g/m$^2$ to 30 g/m$^2$; the basis weight of the second fiber layer is 15 g/m$^2$ to 30 g/m$^2$; the basis weight of the third fiber layer is 10 g/m$^2$ to 30 g/m$^2$; and the basis weight of the wrapping sheet is 35 g/m$^2$ to 60 g/m$^2$.

(7) The wrapping sheet as recited in any one of (1) to (6), in which a pattern is formed in the first fiber layer and/or the second fiber layer by changing the basis weight of the first fiber layer and/or second fiber layer.

(8) The wrapping sheet as recited in any one of (1) to (7), in which the first fiber layer and/or the second fiber layer are colored.

(9) The wrapping sheet as recited in (8), in which the color in which the first fiber layer is colored is different from color in which the second fiber layer is colored.

(10) The wrapping sheet as recited in any one of (1) to (9), in which at least one of the first fiber layer to the three fiber layer includes a sizing agent, and at least partially exhibits a predetermined water resisting property

(11) An individual package of an absorbent article, including: an absorbent article including at least an absorbent exhibiting a fluid-retention property; and a wrapping material wrapping the absorbent article, the wrapping material constituted of the wrapping sheet as recited in any one of claims (1) to (10).

(12) A method of manufacturing a wrapping sheet, including the steps of: forming a first fiber layer from pulp fiber and chemical fiber into a fiber web in a wet paper-forming method, and subsequently by applying a high-pressure water jetting treatment to at least one side of the fiber web to entangle the pulp fiber and the chemical fiber with each other; forming second fiber layer from pulp fiber and chemical fiber; and conveying the first fiber layer, subsequently integrally laminating the first fiber layer and the second fiber layer to each other while the second fiber layer remains wet, thereafter drying the laminated first and second fiber layers.

(13) The method of manufacturing a wrapping sheet as recited in (12), further including the steps of: forming a third fiber layer from pulp fiber and chemical fiber into a fiber web in a wet paper-forming method, and subsequently by applying a high-pressure water jetting treatment to at least one side of the fiber web to entangle the pulp fiber and the chemical fiber with each other; and conveying the first fiber layer and the second fiber layers after integrally laminating the first and second fiber layers to each other while the first and second fiber layers remain wet, subsequently integrally laminating the third fiber layer onto the first and second fiber layers while the third fiber layer remains wet, thereafter drying the laminated first to third fiber layers.

(14) The method of manufacturing a wrapping sheet as recited in any one of (12) and (13), in which, in at least one of the step of forming the first fiber layer and the step of forming the third fiber layer, the fiber web is transferred from a fiber layer forming wire to a patterning wire after the fiber web is formed, and subsequently the high-pressure water jetting treatment is applied to the fiber web thus transferred.

(15) The method of manufacturing a wrapping sheet as recited in any one of (12) to (14), in which, for at least one of the first and third fiber layers, the fiber web is formed by a paper-forming method of any one of a tanmo type and a fourdrinier type.

(16) The method of manufacturing a wrapping sheet as recited in any one of (12), (13) and (15), in which the high-pressure water jetting treatment is applied to the fiber web placed on the fiber layer forming wire.

(17) The method of manufacturing a wrapping sheet as recited in any one of (12) to (16), in which, for the second fiber layer, the fiber web is formed by paper-forming method of any one of a cylinder type and a former type.

According to the present invention, it becomes possible to provide an individual package and a wrapping sheet

EP 2 163 378 B1

which both exhibit a hydrolytic property, a soft tactile impression and appearance, and a high air tightness property which does not allow foreign matters to go in beyond the individual package and the wrapping sheet.

In addition, the present invention employs a wet paper-forming and layer-combining process with a paper machine to manufacture wrapping sheets with a laminated structure. Thus, it makes a secondary process unnecessary, and it achieves cost reduction.

Accordingly, the invention provides a wrapping sheet and a method of manufacturing a wrapping sheet as defined in the appended claims.

BRIEF DESCRIPTION OF THE DRAWINGS

[Fig. 1] Fig. 1 is a perspective view of a first embodiment of a wrapping sheet according to the present invention.

[Fig. 2] Fig. 2 is a perspective view of a second embodiment of the wrapping sheet according to the present invention.

[Fig. 3] Fig. 3 is a plan view showing a first embodiment of an individually wrapped article according to the present invention.

[Fig. 4] Fig. 4 (a) and FigG.4 (b) is a perspective view showing the first embodiment of the individually wrapped article according to the present invention.

[Fig. 5] Fig. 5 is a perspective view of an interlabial pad [Fig. 6] Fig. 6(a) and Fig.6(b) is a perspective view of a second embodiment of the individually wrapped article according to the present invention.

[Fig. 7] Fig. 7 is a schematic view showing a method of manufacturing a wrapping sheet according to the first embodiment of the present invention.

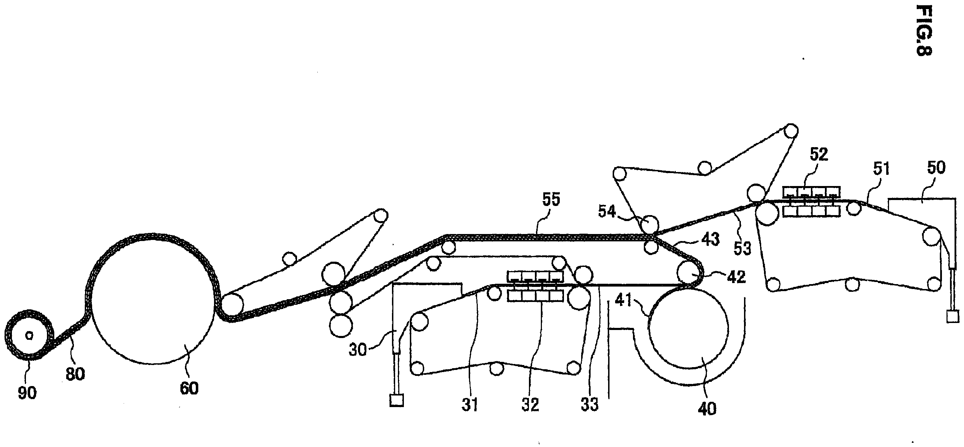

[Fig. 8] Fig. 8 is a schematic view showing a method of manufacturing a wrapping sheet according to the second embodiment of the present invention.

**BEST MODES FOR CARRYING OUT THE INVENTION**

A wrapping sheet according to the present invention is for wrapping an absorbent article including at least an absorbent having a liquid retention property. The wrapping sheet is characterized by including a first fiber layer and a second fiber layer. The first fiber layer includes pulp fiber and chemical fiber, and a high-pressure water jetting treatment is applied to the first fiber layer. The pulp fiber content of the first fiber layer is 30% by mass to 70% by mass. The chemical fiber content of the first fiber layer is 70% by mass to 30% by mass, and the average fiber length of the chemical fiber is 20 mm or less. The second fiber layer is arranged by laminating the second fiber layer to the first fiber layer, and the pulp fiber content of the second fiber layer is 70% by mass to 100% by mass.

Detailed descriptions will be provided hereinbelow for the embodiments of the present invention. The present invention is not limited to the embodiments at all. The present invention can be carried out by applying modifications to the present invention within the scope of the object of the present invention. Duplicated descriptions will be omitted whenever deemed necessary. This omission should not be construed as limiting the present invention.

[Wrapping Sheet]

(First Embodiment)

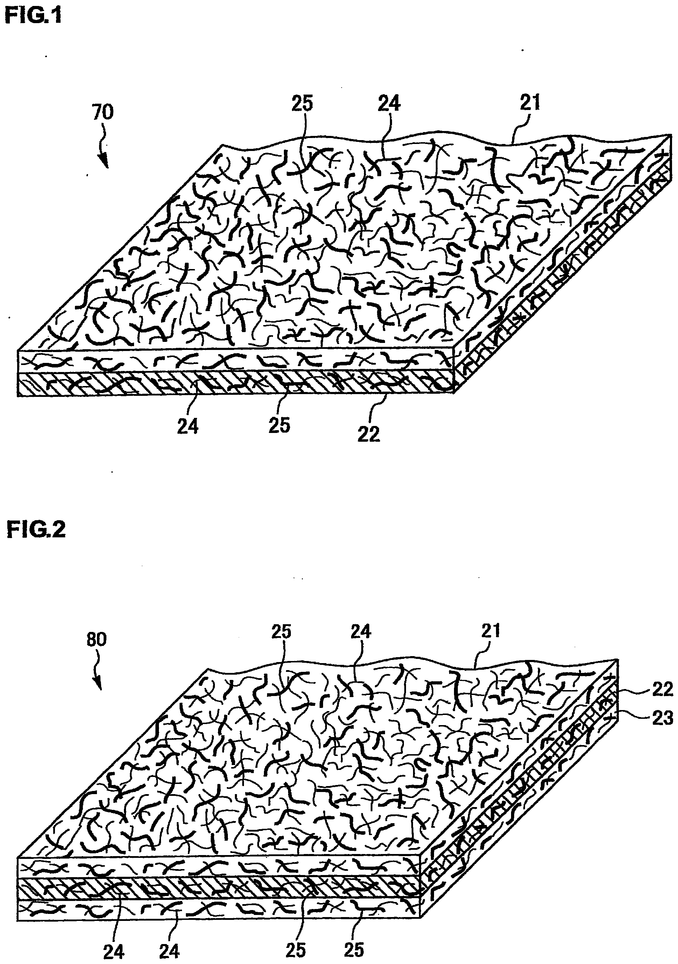

Fig. 1 is a schematic view showing a first embodiment of a wrapping sheet according to the present invention. A two-layered laminate wrapping sheet 70 has a structure in which a first fiber layer 21 and a second fiber layer 22 are laminated to each other. The first fiber layer 21 includes pulp fiber 24 and chemical fiber 25 which are entangled with each other by high-pressure water jetting, which will be described in detail later. The entanglement of the pulp fiber 24 and the chemical fiber 25 by high-pressure water jetting rearranges the pulp fiber 24 and the chemical fiber 25 so that the overall fiber density of the first fiber layer 21 is uneven. This provides the first fiber layer 21 better appearances, and enhances the flexibility of the first fiber layer 21.

The inclusion of the pulp fiber 24 in the first fiber layer 21 increases the strength of the first layer 21.

Various publicly-known pulp fibers can be used as the pulp fiber 24 for the first fiber layer 21. Examples of the publicly-known pulp fibers include LBKP, NBKP, wood pulp, hemp, non-wood pulp such as cotton, regenerated cellulose, acetate fiber, PVA (Polyvinyl Alcohol) fiber, CMC (Carboxyl Methyl Cellulose) fiber. These fibers may be used singly or in combination.

The inclusion of the chemical fiber 25 in the first fiber layer 21 bulks up the first fiber layer 21, and thus imparts flexibility to the first fiber layer 21. This makes it possible to offer the first fiber layers 21 having a soft tactile impression. It is desirable to use the chemical fiber 25 whose fineness is smaller than that of the pulp fiber 24. The use of such a chemical fiber 25 enhances the hydrolytic property and the air tightness property of the first fiber layer 21.

The chemical fiber 25 used in the first fiber layer 21 may be of a synthetic type or of a regenerated type. Various publicly-known chemical fibers can be used as the chemical fiber 25. Examples of the publicly-known chemical fibers include: fibrillated rayon, regenerated cellulose, acryl, polyethylene, acetate, polyolefin, polypropylene, polystyrene, polyester fiber, polyethylene telephthalate, polybutylene telephthalate, polytrimetylene telephthalate and their copolymers; polyamide fiber such as nylon 6 and nylon 6,6; polyacrylonitrile fiber; acrylic fiber; vinylon fiber; splittable fiber; and synthetic pulp. These chemical fibers may be used singly or in combination.

It is desirable that beaten NBKP, beaten LBKP or the like should be used for the purpose of enhancing the air tightness property of the second fiber layer 22.

It is desirable that the pulp fiber 24 content of the first fiber layer 21 as a whole should be 30% by mass to 70% by mass, and that the chemical fiber 25 content of the first fiber layer 21 as a whole should be 70% by mass to 30% by mass. The adoption of such contents causes the first fiber layer 21 to have a higher strength, and makes it possible for the first fiber layer 21 to be easily dispersed by water.

If the pulp fiber 24 content thereof is less than 30% by mass, the smaller pulp fiber content thereof makes the chemical fiber 25 content thereof too large. As a result, the first fiber layer 21 is hard to be dispersed by water. By contrast, if the pulp fiber 24 content thereof is more than 70% by mass, the larger pulp fiber 24 content thereof makes the chemical fiber 25 content thereof too small. As a result, the first fiber layer 21 is hard to be dispersed by water.

If the chemical fiber 25 content of the first fiber layer 21 is less than 30% by mass, the smaller chemical fiber content thereof makes it impossible to obtain a predetermined entanglement strength for the first fiber layer 21 when the chemical fiber 25 and the pulp fiber 24 are entangled by high-pressure water jetting. This makes it difficult to secure the flexibility for the first fiber layer 21. On the other hand, if the chemical fiber 25 content thereof is more than 70% by mass, the larger chemical fiber content thereof makes the entanglement strength too high. This makes the first fiber layer 21 hard to be dispersed by water.

It is desirable that the average fiber length of the chemical fiber 25 in the first fiber layer 21 should be 20 mm or less. By setting the average fiber length of the chemical fiber 25 at 20 mm or less, it is possible to easily disperse the fibers by water flow when the two-layered laminate wrapping sheet 70 is discarded into water. This setting also prevents the formation from being degraded while the layer is manufactured. This setting further prevents the hydrolytic property of the first fiber layer 21 from degrading in a septic tank of, for example, a toilet. After the first fiber layer 21 is hydrolyzed in the septic tank, the setting prevents the first fiber layer 21 from getting entangled with the diffuser tube, and thus to prevent the first fiber layer 21 from hindering the aeration, as well as damaging the diffuser tube.

The basis weight of the first fiber layer 21 can be changed depending on the intended use of the two-layered laminate wrapping sheet 70 whenever deemed necessary. It is desirable, however, that the basis weight of the first fiber layer 21 should be 10 g/m$^2$ to 30 g/m$^2$. The setting of the basis weight of the first fiber layer 21 in this range keeps the first fiber layer 21 strong to a certain degree. If the basis weight of the first fiber layer 21 is less than 10 g/m$^2$, the texture of the first fiber layer 21 may be disturbed while treated by high-pressure water jetting in some cases, and concurrently the first fiber layer 21 may not exert its entanglement effect. On the other hand, if the basis weight of the first fiber layer 21 is more than 30 g/m$^2$, the higher basis weight degrades the hydrolytic property of the first fiber layer 21, and increases the material costs.

Unlike the first fiber layer 21, the second fiber layer 22 can be obtained through a regular forming method without entangling the pulp fiber 24 and the chemical fiber 25 by high-pressure water jetting. The second fiber layer 22 and the first fiber layer 21 are integrally laminated to each other. Because the second fiber layer 22 is made without entangling the pulp fiber 24 and the in the chemical fiber 25 by high-pressure water jetting, the hermetic property of the second fiber layer 22 is enhanced. Thus, the second fiber layer 22 is thus capable of effectively preventing foreign matters from getting in beyond the second fiber layer 22 from outside.

The inclusion of the pulp fibers 24 in the second fiber layer 22 can enhance the strength of the second fiber layer 22.

Various publicly-known pulp fibers can be used as the pulp fibers 24 for the second fiber layer 22. Examples of the publicly-known pulp fibers include LBKP, NBKP, wood pulp, hemp, non-wood pulp such as cotton, regenerated cellulose, acetate fiber, PVA fiber, and CMC fiber. These fibers may be used singly or in combination. In addition, pulp fiber 24 which is the same as that used for the first fiber layer 21 may be used for the second fiber layer 22. Otherwise, pulp fiber 24 different from that used for the first fiber layer 21 may be used for the second fiber layer 22.

It is more desirable that beaten NBKP, beaten LBKP or the like should be used for the purpose of enhancing the hermetic property of the second fiber layer 22.

The inclusion of the chemical fiber 25 in the second fiber layer 22 bulks the second fiber layer 22, and thus imparts flexibility to the second fiber layer 22. This makes it possible to offer the second fiber layer 22 having a soft tactile impression. Furthermore, it is desirable that the chemical fiber 25 whose fineness is smaller than that of the pulp fiber 24 should be used. The use of such a chemical fiber 25 can enhance the hydrolytic property and the hermetic property of the second fiber layer 22.

The chemical fiber 25 used in the second fiber layer 22 may be of a synthetic type or of a regenerated type. Various publicly-known chemical fibers can be used as the chemical fiber 25 for the second fiber layer 22. Examples of the publicly-known chemical fibers include: fibrillated rayon, regenerated cellulose, acryl, polyethylene, acetate, polyolefin, polypropylene, polystyrene, polyester fiber, polyethylene telephthalate, polybutylene telephthalate, polytrimetylene telephthalate and their copolymers; polyamide fiber such as nylon 6 and nylon 6, 6; polyacrylonitrile fiber; acrylic fiber; vinylon fiber; splittable fiber; and synthetic pulp. These chemical fibers may be used singly or in combination. In addition, the chemical fiber 25 which are the same as that used for the first fiber layer 21 may be used for the second fiber layer 22. Otherwise, the pulp fiber 25 different from that used for the first fiber layer 21 may be used for the second fiber layer 22.

It is desirable that the pulp fiber 24 content of the second fiber layer 22 as a whole should be 70% by mass to 100% by mass, and that the chemical fiber 25 content of the second fiber layer 22 as a whole should be 30% by mass or less. The adoption of such contents increases the air-flow resistance value and enhances the hermetic property of the second fiber layer 22. This makes it possible for the second fiber layer 22 to effectively prevent foreign matters from getting in beyond the second fiber layer 22 from outside.

If the pulp fiber 24 content thereof is less than 70% by mass, the smaller pulp fiber content thereof makes the chemical fiber 25 content thereof too large. As a result, the second fiber layer 22 is hard to hydrolyze. By contrast, if the chemical fiber 25 content thereof is more than 30% by mass, the larger chemical fiber content thereof causes troubles on the production process while the second fiber layer 22 is manufactured. Examples of the troubles include: increasing the denseness; degrading the water-filtering property; and reducing the fiber yields. In addition, the larger chemical fiber content thereof hardens the second fiber layer 22 too much. As a result, for example, the second fiber layer 22 becomes apt to get creased while used as an individual-wrapping material. Furthermore, the larger chemical pulp content thereof may increase the material costs in some cases.

It is desirable that the average fiber length of the chemical fiber 25 in the second fiber layer 22 should be 20 mm or less. The setting of the average fiber length of the chemical fiber 25 at 20 mm or less makes it possible to disperse the fibers by water easily when the two-layered laminate wrapping sheet 70 is discarded into water. This setting also prevents the formation from being degraded while the second fiber layer 22 is manufactured. This setting further prevents the hydrolytic property of the second fiber layer 22 from degrading in a septic tank of, for example, a toilet. After the second fiber layer 22 is hydrolyzed in the septic tank, too, the setting prevents the fibers from getting entangled with the diffuser tube, and thus to prevent the fibers from hindering the aeration, as well as preventing the fibers from damaging the diffuser tube.

The basis weight of the second fiber layer 22 can be changed depending on the intended use of the two-layered laminate wrapping sheet 70 whenever deemed necessary. It is desirable, however, that the basis weight of the second fiber layer 22 should be 15 g/m$^2$ to 30 g/m$^2$. The setting of the basis weight of the second fiber layer 22 in this range keeps the second fiber layer 22 strong to a certain degree. If the basis weight of the second fiber layer 22 is less than 15 g/m$^2$, the smaller basis weight may degrade the hermetic property of the second fiber layer 22 in some cases. On the other hand, if the basis weight of the second fiber layer 22 is more than 30 g/m$^2$, the larger basis weight hardens the second fiber layer 22, causes the sheet to make sounds, makes the sheet crease easily, and deteriorates the tactile impression.

The basis weight of the two-layered laminate wrapping sheet 70 can be changed depending on the intended use of the two-layered laminate wrapping sheet 70 whenever deemed necessary. It is desirable, however, that the basis weight of the two-layered laminate wrapping sheet 70 should be 25 g/m$^2$ to 60 g/m$^2$. If the basis weight of the two-layered laminate wrapping sheet 70 is less than 25 g/m$^2$, the smaller basis weight may degrade the hermetic property of the two-layered laminate wrapping sheet 70 in some cases. On the other hand, if the basis weight of the two-layered laminate wrapping sheet 70 is more than 60 g/m$^2$, the larger basis weight degrades the hydrolytic property of the two-layered laminate wrapping sheet 70.

It is desirable that the dry strength of the two-layered laminate wrapping sheet 70 should be 2. ON/25 mm or more in the machine direction MD. It is more desirable that the dry strength thereof should be 5.0N/25 mm in the machine direction MD. In addition, it is desirable that the dry strength of the two-layered laminate wrapping sheet 70 should be 2.0N/25 mm or more in the cross direction CD. It is more desirable that the dry strength thereof should be 2.5N/25 mm or more in the cross direction CD. The setting of the dry strengths thereof in such ranges makes it possible for the two-layered laminate wrapping sheet 70 to maintain its required strengths. Moreover, it is desirable that the wet strength of the two-layered laminate wrapping sheet 70 should be 2. ON/25 mm or more both in the machine direction MD and in the cross direction CD. The setting of the wet strengths thereof in such ranges makes it possible for the two-layered laminate wrapping sheet 70 to maintain its required strengths even while in a wet condition.

Once the two-layered laminate wrapping sheet 70 is discarded into a toilet bowl or the like, the pulp fiber 24 included in the first fiber layer 21 and the second fiber layer 22 immediately starts to be dissolved by the water flow of a large amount of water in the toilet bowl. In the first fiber layer 21, once the pulp fiber 24 starts to be dissolved, the chemical fiber 25 which is entangled with the pulp fiber 24 starts to be dissolved into the water. The untangling of the pulp fibers 24 and the chemical fiber 25 quickly disperses the fibers.

The hydrolytic property of the two-layered laminate wrapping sheet 70 can be changed depending on the intended use and size of the two-layered laminate wrapping sheet 70 whenever deemed necessary. Take a case where, for example, the two-layered laminate wrapping sheet 70 is a squared sheet with a size of 10cmx10cm. When the two-layered laminate wrapping sheet 70 is shaken at a shaking speed of 240 rpm for 30 minutes with the two-layered laminate wrapping sheet 70 being placed in 800ml of distilled water, it is desirable that the two-layered laminate wrapping sheet 70 should be so dispersed into water that even the largest remaining piece of the two-layered laminate wrapping sheet 70 becomes 50 $cm^2$ or less in size. It is more desirable that the two-layered laminate wrapping sheet 70 should be so dispersed into water that even the largest remaining piece thereof becomes 25 $cm^2$ or less in size. It is far more desirable that the two-layered laminate wrapping sheet 70 should be so dispersed into water that no trace of the original form thereof remains. If the two-layered laminate wrapping sheet 70 is so dispersed into water that the largest remaining piece thereof is still not less than 50 $cm^2$ in size, in some cases, the largest remaining piece thereof may clog the toilet drain or the like when the two-layered laminate wrapping sheet 70 is discarded by toilet flushing or the like.

A pattern may be formed in the first fiber layer 21 and the second fiber layer 22 by changing the basis weight of each of the first fiber layer 21 and the second fiber layer 22, depending on the necessity, to such an extent that the hydrolytic properties thereof are maintained.

At least one part of the first fiber layer 21 and/or the second fiber layer 22 may be designed to have a predetermined water resisting property by including a sizing agent therein. A rosin derivative, AKD (Alkyl Ketene Dimer) and ASA (Alkenyl Succinic Anhydride) can be cited as examples of the sizing agent.

The first fiber layer 21 and/or the second fiber layer 22 may be colored to such an extent that the hydrolytic property thereof is maintained depending on the necessity. Various publicly-known adhesion promoters can be used as the adhesion promoter for the color development. Examples of the usable adhesion promoters include: common direct cationic dyes such as CI. Direct Red 81; and cationic dyes each including a sizing agent. It should be noted that the first fiber layer 21 and the second fiber layer 22 may be colored in different colors.

In addition, depending on the necessity, the two-layered laminate wrapping sheet 70 can be turned into a heat sealable one by blending a thermally-adhesive fiber, which does not develop its thermal adhesiveness at a drying temperature used when the chemical fiber 25 is manufactured, into the first fiber layer 1 and the second fiber layer 2. In this case, if the average fiber length of the thermally-adhesive fiber is set at 20 mm or less, it is possible to prevent the hydrolytic property thereof from being degraded. For this reason, the two-layered laminate wrapping sheet 70 into which the thermally-adhesive fiber is blended can exhibit the heat-seal property, the air tightness property and the hydrolytic property at the same time, because part of the two-layered laminate wrapping sheet 70 which is not heat-sealed is capable of maintaining its hydrolytic property fully.

In the case where the two-layered laminate wrapping sheet 70 is discarded into a large amount of water in a flush toilet, and the like, first of all, the second fiber layer 22 is hydrolyzed, and the hydrolysis of the first fiber layer 22 is thus accelerated. Thereby, the two-layered laminate wrapping sheet 70 is hydrolyzed.


(Second Embodiment)


A second embodiment of the wrapping sheet according to the present invention is the same as the first embodiment thereof except that, as shown in Fig. 2, a third fiber layer 23 is arranged on a side of the second fiber layer 22 which does not oppose to the first fiber layer 21, and the first to third fiber layers 21, 22 and 23 are laminated one to another. Like the first fiber layer 21, the third fiber layer 23 includes the pulp fiber 24 and the chemical fiber 25 which are entangled with each other. These two fibers are obtained by entangling them with each other by high-pressure water jetting treatment. The entanglement by high-pressure water jetting treatment rearranges the pulp fiber 24 and the chemical fiber 25 so that the overall fiber density of the third fiber layer 23 is uneven. This provides the third fiber layer 23 better appearances, and enhances the flexibility of the third fiber layer 23.

The inclusion of the pulp fiber 24 in the third fiber layer 23 increases the strength of the third layer 23.

Various publicly-known pulp fibers can be used as the pulp fiber 24 for the third fiber layer 23. Examples of the publicly-known pulp fibers include LBKP, NBKP, wood pulp, hemp, non-wood pulp such as cotton, regenerated cellulose, acetate fiber, PVA (Polyvinyl Alcohol) fiber, CMC (Carboxyl Methyl Cellulose) fiber. These fibers may be used singly or in combination. The pulp fiber 24 used for the third fiber layer 23 may be the same as the pulp fiber 24 used for the first layer 21. Otherwise, the pulp fiber 24 used for the third fiber layer 23 may be different from the pulp fiber 24 used for the first fiber layer 21.

The inclusion of the chemical fiber 25 in the third fiber layer 23 bulks up the third fiber layer 23, and thus imparts flexibility to the third fiber layer 23. This makes it possible to offer the third fiber layers 23 having a soft tactile impression. It is desirable to use the chemical fiber 25 whose fineness is smaller than that of the pulp fiber 24. The use of such a chemical fiber 25 enhances the hydrolytic property and the air tightness property of the third fiber layer 23.

The chemical fiber 25 used in the third fiber layer 23 may be of a synthetic type or of a regenerated type. Various publicly-known chemical fibers can be used as the chemical fiber 25 for the first fiber layer 21. Examples of the publicly-known chemical fibers include: fibrillated rayon, regenerated cellulose, acryl, polyethylene, acetate, polyolefin, polypropylene, polystyrene, polyester fiber, polyethylene telephthalate, polybutylene telephthalate, polytrimetylene telephthalate and their copolymers; polyamide fiber such as nylon 6 and nylon 6, 6; polyacrylonitrile fiber; acrylic fiber; vinylon fiber; splittable fiber; and synthetic pulp. These chemical fibers may be used singly or in combination. The chemical fiber 25 used for the third fiber layer 23 may be the same as the chemical fiber 25 used for the first layer 21. Otherwise, the chemical fiber 25 used for the third fiber layer 23 may be different from the chemical fiber 25 used for the first fiber layer 21.

It is desirable that beaten NBKP, beaten LBKP or the like should be used for the purpose of enhancing the air tightness property of the second fiber layer 22.

It is desirable that the pulp fiber 24 content of the third fiber layer 23 as a whole should be 30% by mass to 70% by mass, and that the chemical fiber 25 content of the third fiber layer 23 as a whole should be 70% by mass to 30% by mass. The adoption of such contents causes the third fiber layer 23 to have a higher strength, and makes it possible for the third fiber layer 23 to be easily dispersed by water. The pulp fiber 24 content and the chemical fiber 25 content of the third fiber layer 23 may be the same as the pulp fiber 24 content and the chemical fiber 25 content of the first fiber layer 21, respectively. Otherwise, pulp fiber 24 content and the chemical fiber 25 content of the third fiber layer 23 may be different from the pulp fiber 24 content and the chemical fiber 25 content of the first fiber layer 21.

If the pulp fiber 24content thereof is less than 30% by mass, the smaller pulp fiber content thereof makes the chemical fiber 25 content thereof too large. As a result, the third fiber layer 23 is hard to hydrolyze. By contrast, if the pulp fiber 24 content thereof is more than 70% by mass, the larger pulp fiber 24 content thereof makes the chemical fiber 25 content thereof too small. As a result, the third fiber layer 23 is hard to be dispersed by water.

If the chemical fiber 25 content of the third fiber layer 23 is less than 30% by mass, the smaller chemical fiber content thereof makes it impossible to secure a predetermined entanglement strength for the third fiber layer 23 when the chemical fiber 25 and the pulp fiber 24 are entangled by high-pressure water jetting treatment. This makes it difficult to secure the flexibility for the third fiber layer 23. On the other hand, if the chemical fiber 25 content thereof is more than 70% by mass, the larger chemical fiber content thereof makes the entanglement strength too high. This makes the first fiber layer 21 hard to hydrolyze easily.

It is desirable that the average fiber length of the chemical fiber 25 in the third fiber layer 23 should be 20 mm or less. By setting the average fiber length of the chemical fiber 25 at 20 mm or less, it is possible to easily disperse the fibers by water flow when the three-layered laminate wrapping sheet 80 is discarded into water. This setting also prevents the formation from being degraded while the third fiber layer 23 is manufactured. This setting further prevents the hydrolytic property of the third fiber layer 23 from degrading in a septic tank of, for example, toilet. After the third fiber layer 23 is hydrolyzed in the septic tank, the setting prevents the fibers from getting entangled with the diffuser tube, and thus to prevent the fibers from hindering the aeration, as well as preventing the fibers from damaging the diffuser tube.

The basis weight of the third fiber layer 23 can be changed depending on the intended use of the three-layered laminate wrapping sheet 80 whenever deemed necessary. It is desirable, however, that the basis weight of the third fiber layer 23 should be 10 g/m$^2$ to 30 g/m$^2$. The setting of the basis weight of the third fiber layer 23 in this range keeps the third fiber layer 23 strong to a certain degree. If the basis weight of the third fiber layer 23 is less than 10 g/m$^2$, the texture of the third fiber layer 23 may be disturbed while treated by high-pressure water jetting in some cases, and concurrently the third fiber layer 23 may not exert its entanglement effect. On the other hand, if the basis weight of the third fiber layer 23 is more 30 g/m$^2$, the higher basis weight degrades the hydrolytic property of the third fiber layer 23, and increases the material costs. The basis weight of the third fiber layer 23 may be the same as that of the first fiber layer 21, or may be different from that of the first fiber layer 21.

In addition, the basis weight of the three-layered laminate wrapping sheet 80 can be changed depending on the intended use of the three-layered laminate wrapping sheet 80 whenever deemed necessary. It is desirable that the basis weight thereof should be 35 g/cm$^2$ to 60 g/cm$^2$. If the basis weight thereof is less than 35 g/m$^2$, the smaller basis weight thereof may degrade the air tightness property of the three-layered laminate wrapping sheet 80 in some cases. By contrast, if the basis weight thereof is more than 60 g/cm$^2$, the larger basis weight thereof degrades the hydrolytic property of the three-layered laminate wrapping sheet 80.

It is desirable that the dry strength of the three-layered laminate wrapping sheet 80 should be 2.0 N/25 mm or more in the machine direction MD. It is more desirable that the dry strength thereof should be 5.0 N/25 mm in the machine direction MD. In addition, it is desirable that the dry strength of the three-layered laminate wrapping sheet 80 should be 2.0 N/25 mm or more in the cross direction CD. It is more desirable that the dry strength thereof should be 2.5 N/25 mm or more in the cross direction CD. The setting of the dry strengths thereof in such ranges makes it possible for the three-layered laminate wrapping sheet 80 to maintain its required strengths. Moreover, it is desirable that the wet strength of the three-layered laminate wrapping sheet 80 should be 2.0 N/25 mm or more both in the machine direction MD and in the cross direction CD. The setting of the wet strengths thereof in such ranges makes it possible for the three-layered laminate wrapping sheet 80 to maintain its required strengths even while in a wet condition.

Once the three-layered laminate wrapping sheet 80 is discarded into a toilet bowl or the like, the pulp fiber 24 included in the first fiber layer 21 to the third fiber layers 23 immediately starts to be dissolved by the water flow of a large amount of water in the toilet bowl. Once the pulp fiber 24 in the first fiber layer 21 and the third fiber layer 23 starts to be dissolved, the chemical fiber 25 which is entangled with the pulp fiber 24 starts to be dissolved into the water. The untangling of the pulp fibers 24 and the chemical fiber 25 quickly disperses the fibers.

The hydrolytic property of the three-layered laminate wrapping sheet 80 can be changed depending on the intended use and size of the three-layered laminate wrapping sheet 80 whenever deemed necessary. Take a case where, for example, the three-layered laminate wrapping sheet 80 is a squared sheet with a size of 10cmx10cm. When the three-layered laminate wrapping sheet 80 is shaken at a shaking speed of 240 rpm for 30 minutes with the sheet being placed in 800ml of distilled water, it is desirable that the three-layered laminate wrapping sheet 80 should be so dispersed into water that even the largest remaining piece of the three-layered laminate wrapping sheet 80 becomes 50 cm$^2$ or less in size. It is more desirable that the three-layered laminate wrapping sheet 80 should be so dispersed into water that even the largest remaining piece thereof becomes 25 cm$^2$ or less in size. It is far more desirable that the three-layered laminate wrapping sheet 80 should be so dispersed into water that no trace of the original form thereof remains. If the three-layered laminate wrapping sheet 80 is so dispersed into water that the largest remaining piece thereof is still not less than 50 cm$^2$ in size, in some cases, the largest remaining piece thereof may clog the toilet drain or the like when the three-layered laminate wrapping sheet 80 is discarded by toilet flushing or the like.

The three-layered laminate wrapping sheet 80 can be turned into a heat sealable one by blending a thermally-adhesive fiber, which does not develop its thermal adhesiveness at a drying temperature used when the chemical fiber 25 is manufactured, into the first fiber layer 21 and the third fiber layer 23 depending on the necessity. In this case, if the average fiber length of the thermally-adhesive fiber is set at 20 mm or less, it is possible to prevent the hydrolytic property thereof from being degraded. For this reason, the three-layered laminate wrapping sheet 80 into which the thermally-adhesive fiber is blended can exhibit the heat-seal property, the air tightness property and the hydrolytic property at the same time, because part of the three-layered laminate wrapping sheet 80 which is not heat-sealed is capable of maintaining its hydrolytic property fully.

In a case where the three-layered laminate wrapping sheet 80 is discarded into a large amount of water in a flush toilet, and the like, first of all, the second fiber layer 22 is hydrolyzed, and the first fiber layer 21 to the third fiber layer 23 are thus separated from each other. The layer separation accelerates the hydrolysis of the first fiber layer 21 and third fiber layer 23. Finally, the three-layered laminate wrapping sheet 80 is hydrolyzed.

[Individual Package]

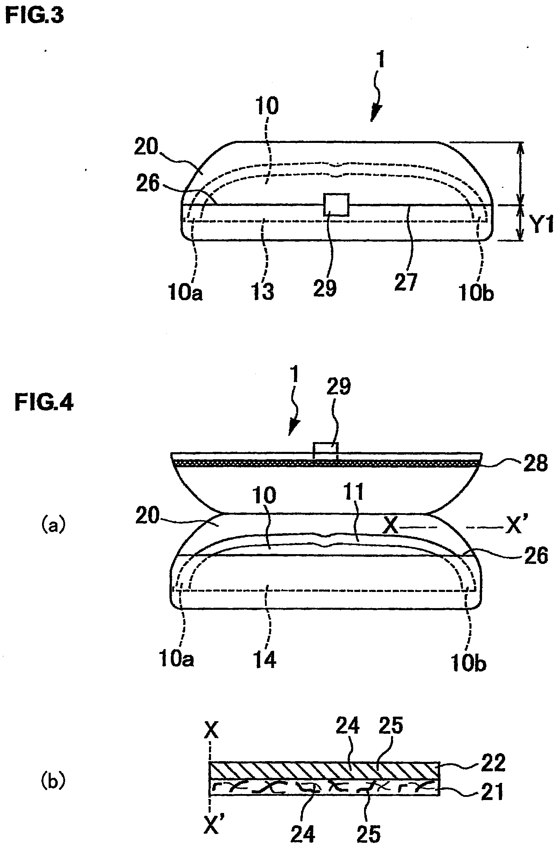

Figs. 3 and 4 are schematic view showing an outline of an individual package 1 according to the first embodiment. As shown in Figs. 3 and 4, the individual package 1 includes an interlabial pad 10 and a wrapping material 20 wrapping the interlabial pad 10. For explanatory convenience, the absorbent article is explained as being the interlabial pad 10. However, the absorbent article is not limited to the interlabial pad 10, and includes a sanitary napkin and a pantiliner.

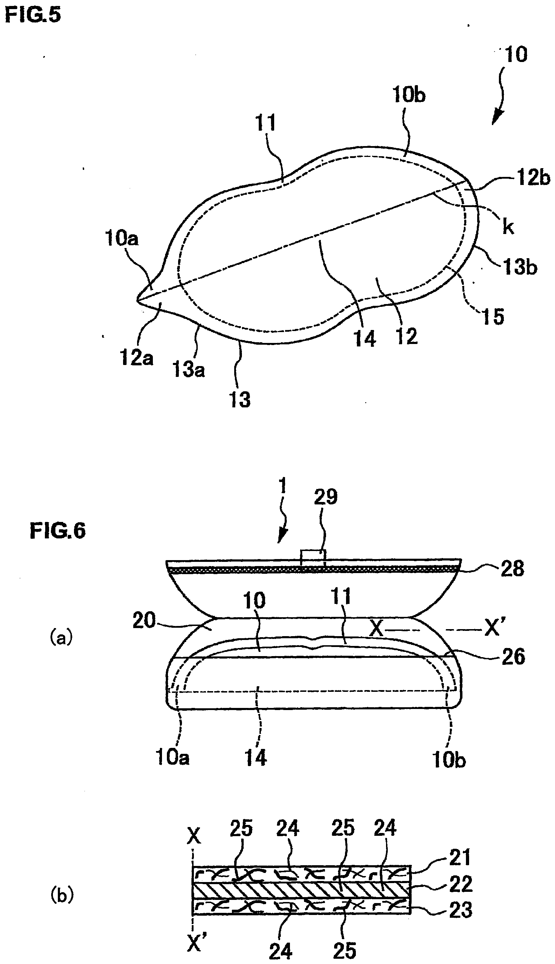

Fig. 5 shows a perspective view of the interlabial pad 10 as the absorbent article. The interlabial pad 10 is folded into halves along the center axis which extends in the longitudinal direction. The interlabial pad 10 is worn with at least a part of the pad being interposed between the minor labia in the labia in a way that at least one part of the pad near the center axis contacts the vestibular floor in the labia.

The interlabial pad 10 is worn with a part of the pad being interposed between the labia of the wearer. As shown in Fig. 5, the interlabial pad 10 includes: a fluid-permeable top sheet 12, which is a covering material, and which exhibits a fluid permeability property; a fluid-impermeable back sheet 13, which is a covering material, and which exhibits a fluid-impermeability property which virtually does not allow any fluid to permeate the back sheet; and an absorbent 15, which is arranged between the fluid-permeable top sheet 12 and the fluid-impermeable back sheet 13, and which exhibits a fluid retention property. In addition, the interlabial pad 10 includes a peripheral part 11 constituting the border of the interlabial pad 10. The peripheral part 11 includes a front end part 10a and a rear end part 10b which are located in the two ends of the interlabial pad 10 in the longitudinal direction thereof. Furthermore, the interlabial pad 10 is folded into halves along the center line k in the longitudinal direction thereof with the folded halves of the fluid-impermeable back sheet 13 being lied over one another when the interlabial pad 10 is worn. Moreover, the interlabial pad 10 includes a vestibular floor touching area 14 which contacts the vestibular floor of a wearer when the interlabial pad 10 is worn.

The fluid-permeable top sheet 12 and the fluid-impermeable back sheet 13 both for covering an absorbent member 15 includes: a fluid-permeable top sheet front end part 12a and a fluid-impermeable back sheet front end part 13a both facing the pubis while the interlabial pad 10 is worn; and a fluid-permeable top sheet rear end part 12b and a fluid-impermeable back sheet rear end part 13b both situated to the buttocks while the pad 10 is worn. In the case of the present embodiment, the fluid-permeable top sheet front end part 12a and the fluid-impermeable back sheet front part 13a constitute the front end part 10a of the peripheral part 11 of the interlabial pad 10, and fluid-permeable the top sheet rear end part 12b and the fluid-impermeable back sheet rear end part 13b constitute the rear end part 10b of the peripheral part 11 thereof.

The interlabial pad 10 is formed in a shape such as oval, gourd-shaped, teardrop-shaped and the like, so that the interlabial pad 10 fit to the female labia. With regard to the external dimensions of the interlabial pad 10, it is desirable that the width of the interlabial pad 10 in the center axial direction thereof should be 40 mm to 180 mm. It is more desirable that the width thereof in the center axial direction should be 80 mm to 120 mm. In addition, it is desirable that the width of the interlabial pad 10 in a direction perpendicular to the center axis should be 20 mm to 100 mm. It is more desirable that the width thereof in the perpendicular direction should be 50 mm to 80 mm. It should be noted that the width thereof in the direction perpendicular to the center axis is the width of the interlabial pad 10 which is flat before folded into halves. When the interlabial pad 10 is worn while folded in halves, the dimension of the interlabial pad 10 in the perpendicular direction should be almost a half of the desirable width. As described above, the interlabial pad 10 is basically of a type which is interposed between the labia with the flat pad being folded into halves. Instead, however, a bar-like shape and a cylinder-like shape may be adopted for the interlabial pad 10. The width of the pad other than the type which is worn with the flat pad being folded in halves, the width of the pad in the direction perpendicular to the center axis should be almost a half of the above-described desirable width.

A survey conducted by the applicants concludes: an average length of the cunnus (from the anterior labial commissure to the posterior labial commissure) is approximately 80 mm; the average distance from the clitoris to the ostium vaginae is approximately 40 mm; the distance from the ostium vaginae to the anus is approximately 45 mm. For the purpose of preventing the menstrual blood from flowing out, it is desirable that the front edge end portion of the interlabial pad 10 should cover up to at least the clitoris. For the purpose of preventing the pad from coming out of place due to movement of the muscles of the buttocks, it is desirable that the rear edge end portion of the interlabial pad 10 should not reach the anus. For the purpose of satisfying these conditions, it is desirable that the external dimensions of the interlabial pad 10 should be within the foregoing ranges.

It is desirable that the absorbent article according to the present invention should be made of a water-dispersible material, a biodegradable material and a water-soluble material. This is because the below-described wrapping material 20 is water-degradable. After used, the absorbent article thus made can be discarded into the toilet. Thus, making it possible to discard the absorbent article conveniently and cleanly, and reduce the amount of garbage in the toilet.

"Water-disperible" is synonymous to water-degradable. "Water-dispersible" is used to describe a property which does not allow a limited small amount of moisture or menstrual blood to affect a product while the product is in use, but which causes ingredient fibers of the product to be dispersed in a large amount of water or water flow within a range that the dispersed fibers do not clog at least a drainage pipe of a regular toilet.

"Biodegradable" is used to describe a property which causes ingredient substances constituting a product to be degraded into gases such as carbon dioxide and methane, water, and biomass through a natural ecological process in the environment in which bacteria including actinomycetes, and other microorganisms exist. In addition, biodegradability (or biodegradation speed, and degree of biodegradation) of the product is correspondingly treated as that of a natural material such as fallen leaves, and as a synthetic polymer recognized as being biodegradable.

"Water-soluble" is used to describe a property which does not allow a limited small amount of moisture or menstrual blood to affect a product while the product is in use, but which causes the product to be dissolved in a large amount of water or water flow.

As shown in Figs. 3 and 4, the wrapping material 20 contains the whole interlabial pad 10 with the pad being wrapped with the wrapping material 20. The wrapping material includes an unsealed opening 26. A part of the wrapping material 20 overlaps another part of the wrapping material 20 in the vicinity of unsealed opening 26 . The overlapping parts of the wrapping material 20 are adhered to each other to be able to unseal and detach.

The wrapping material 20 shown in Fig. 3 is provided with a protective area Y1 which has a higher rigidity than that of the peripheral part 11 of the interlabial pad 10. The width dimension of the protective area Y1 is large enough to cover the vestibular floor touching area 14, in other words covers 5% to 70% of the width of the interlabial pad 10 while in the state of being wrapped with the wrapping material 20. The protective area Y1 is unintermittently provided to the wrapping material 20 in the longitudinal direction. An embossing seal 28, for connecting a part of the wrapping material 20 to another part of the wrapping material 20 is provided to an end side of the wrapping material in the longitudinal direction of the wrapping material 20. The rigidity of the protective area Y1 can be increased by this embossing seal 28, as well. An adhesive is provided in the longitudinal direction between the overlapping parts of the wrapping material 20 for the purpose of increasing the air-tightness of the wrapping material 20. This adhesive increases the rigidity of the wrapping material 20. For this reason, it is possible for the vestibular floor touching area 14 to keep its original shape even if an external pressure is applied to the individual package 1 while the individual package 1 is being carried. In addition, the individual package 1 is a package of the interlabial pad 10 hermetically sealed with the wrapping material 20 with the air being filled inside. For this reason, even if the individual package 1 is dropped, not only the peripheral part 11 but also the other parts of the interlabial pad 10 inside the individual package 1 are prevented from being damaged.

The embossing seal 28, for connecting a part of the wrapping material 20 to another part of the wrapping material 20 constituting the wrapping material 20,may be provided to the entire peripheral portion of the wrapping material 20 additionally. Furthermore, an unsealing part 27 configured by the overlapping parts of the wrapping material 20 may be provided with an adhesive tying tape 29 for the purpose of increasing the unsealability of the unsealing part 27.

The front end part 10a of the peripheral part 11 of the interlabial pad 10 tends to come into contact with the clitoris of a wearer while the interlabial pad 10 is worn. The rear end part 10b thereof tends to come into contact with the anus of the wearer while the interlabial pad 10 is worn. For this reason, additional protective areas Y1 are provided to the wrapping material 20 depending on the necessity. This prevents the front end part 10a and the rear end part 10b from warping. As a result, it is possible to prevent the font end part 10a and the rear end part 10b from hurting the clitoris and anus of the wearer, respectively.

The protective area Y1 may be provided in order to protect the entire peripheral part 11 of the interlabial pad 10. Otherwise, the protective area Y1 may be provided in order to protect the vestibular floor touching area 14 only. Otherwise, the protective area Y1 may be provided in order to protect the front end part 10a (including the fluid-permeable top sheet front end part 12a and the fluid-impermeable back sheet front end part 13a) and the rear end part 10b (including the fluid-permeable top sheet rear end part 12b and the fluid-impermeable back sheet rear end part 13b) only.

It is desirable that the buckling strength of the protective area Y1 should be 100 mN or more, but 2000 mN or less. The buckling strength within this range prevents the peripheral part 11 from warping during a wrapping process, or while the individual package 1 is being carried. In addition, it is desirable that the buckling strength of the peripheral part 11 of the interlabial pad 10 should be 50 mN or more, but 400 mN or less.

The wrapping material 20 is the two-layered laminate wrapping sheet 70, which has a configuration in which the first fiber layer 21 is arranged in the outer side of the individual package 1 as shown by the cross-section taken along the X-X' line of Fig. 4. The use of the two-layered laminate wrapping sheet 70 as the wrapping material 20 provides the wrapping material 20 better appearance and more flexible. In stead of the two-layered laminate wrapping sheet 70,as shown in FIG.6, the three layered laminate wrapping sheet 80 may be used as the wrapping material 20.

[Method of Manufacturing a Wrapping Sheet]

A method of manufacturing a wrapping sheet according to the present invention is characterized by including: a step of forming a first fiber layer 21 by forming pulp fiber 24 and chemical fiber 25 into a fiber web by a wet forming method, and subsequently by applying a high-pressure water jetting treatment to the two sides or one side of the fiber web to entangle the pulp fiber 24 and the chemical fiber 25; a step of forming pulp fiber 24 and chemical fiber 25 into a second fiber layer 22; and a step of conveying the first fiber layer 21, and integrally laminating the second fiber layer 22 to the first fiber layer 21.

(Method of Manufacturing a Two-layered Laminated Wrapping Sheet 70)

First of all, a material liquid dispersed in water with a predetermined concentration is sent to a first web former 30 and a second web former 40, and the material liquid thus sent is made into a first web 31 and a second web 41. It should be noted that, in a case where the first fiber layer 21 and the second fiber layer 22 are intended to be colored, a sizing agent, a cationic sizing agent, a cationic dye or the like may be added to the material liquid.

The first fiber layer 21 is formed by forming the pulp fiber 24 and the chemical fiber 25 into the fiber web (the first web 31) by the wet forming method, and thereafter by applying the high-pressure water jetting treatment to the first web 31. In this respect, the fiber web means a sheet-shaped fiber mass in which most ingredient fibers are oriented in the same direction. The fiber web may be made by a dry forming method instead of the wet forming method. For this high-pressure water jetting treatment, a high-pressure water jetting machine of a generally-used type is employed.

When the high-pressure water jetting treatment is applied to either or both sides of the fiber web (the first web 31) by use of a water jet with a relative high pressure, the pulp fiber 24 and the chemical fiber 25 are strongly entangled to each other in parts of the first web where the water jet is applied, whereas the two fibers are kept bulked up in the other parts of the first web where the water jet is not applied. Consequently, in the first fiber layer 21 the pulp fiber 24 and the chemical fiber 25 are highly integrally entangled to each other, and are bulked up, as well as. This exhibits a better air permeability, water absorbing property, heat retaining property, softness and the like.

The high-pressure water jetting treatment involves applying a jet of high-pressure water to the pulp fiber 24 and the chemical fiber 25, and thereby entangling parts of the two fibers 24 and 25, and thus combining the jet-applied parts thereof together. An apparatus and conditions employing a regular technology for manufacturing a non-woven fabric can be used as an apparatus and conditions for the high-pressure water jetting treatment.

Detailed descriptions will be herein provided for the high-pressure water jetting treatment. A fiber web is placed on a conveyer belt continuously running, and a jet of high-pressure water is applied to the fiber web placed thereon in a way that the jet passes the fiber web from its front surface through its back surface. In the case of this high-pressure water jetting treatment, properties of the obtainable first fiber layer 21 can be changed by changing the weighing capacity of the fiber web, the pore size of the jet nozzle, the number of pores in the jet nozzle, the work load (or energy) supplied for treating the fiber web and the like whenever deemed necessary.

In the case of the present embodiment, it is desirable that the high-pressure water jetting treatment should be applied to the fiber web (or the first web 31) in a way that the work load calculated by use of Equation (1) is 0.05 kW/m$^2$ to 0.5 kW/m$^2$ for each treatment of one surface of the fiber web (or the first web 31) . If the work load is less than 0.05 kW/m$^2$, the smaller work load decreases the bulk of the first fiber layer 21. By contrast, if the work load is more than 0.5 kW/m$^2$, the larger work load may entangle the fibers with each other too much so that the hydrolytic property of the first fiber layer 21 is degraded, or may destroy the fiber web. This high-pressure water jetting treatment can be applied to either or both sides of the fiber web. If, for example, the high-pressure water jetting treatment with a work load of 0.05 kW/m$^2$ to 0.5 kW/m$^2$ is applied to either or both sides of the fiber web once to 6 times, the first fiber layer 21 can be obtained with its desirable hydrolytic property and wet strength.

[Formula 1]

$$\text{Work load (kW/m}^2)$$
$$= [1.63 \times \text{jet pressure (kgf/cm}^2) \times \text{jet amount (m}^3/\text{min)}]$$
$$\div \text{treatment rate (m/min)} \qquad \dots \text{Equation (1)}$$

In the case where the work load is 0. 05 kW/m$^2$ to 0. 5 kW/m$^2$, the high-pressure water jetting treatment can be applied thereto with a condition that, for example, the pore size of the nozzle is 90 $\mu$m to 100 $\mu$m, and the pores are arranged in the nozzle at intervals of 0. 3 mm to 2.0 mm in the cross direction CD. In this case, the fibers are appropriately entangled with each other.

After the fiber web (or the first web 31) is made, the high-pressure water jetting treatment may be applied to the fiber web without drying the fiber web. Otherwise, the high-pressure water jetting treatment may be applied thereto after drying the fiber web.

The high-pressure water jetting treatment may be applied to the formed first web 31 which is conveyed to a high-pressure water jetting treatment unit 32 while placed on a fiber layer forming wire directly. Otherwise, depends on the necessity, the high-pressure water jetting treatment may be applied to the first web 31 which is conveyed to a high-pressure water jetting treatment unit 32 after transferred from the fiber layer forming wire to a patterning wire.

It is desirable that a web forming machine of an inclined tanmo or fourdrinier type which is capable of easily distributing even long ingredient fibers randomly should be used as the first web former 30.

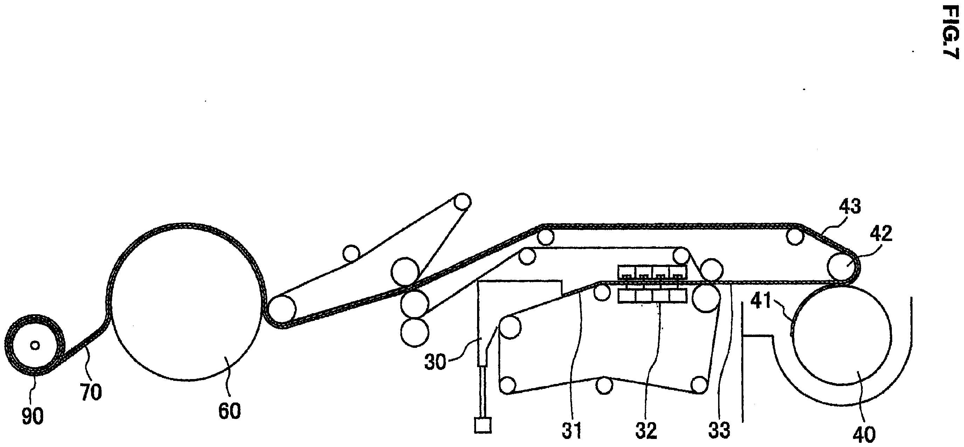

The first high-pressure water jetting unit 32 may have a configuration in which the first high-pressure water jetting unit 32 uses a web forming net (not illustrated) with the first web former 30, and in which a high-pressure water jetting nozzle apparatus (not illustrated) is arranged above the web forming net. Furthermore, in a case where a patterned sheet is intended to be obtained through the high-pressure water jetting treatment, it is desirable that the high-pressure water jetting treatment should be applied to the first web 31 by conveying the first web 31 to the first high-pressure water jetting treatment unit 32 which is arranged in the back of the first web former 30 as shown in Fig. 7.

The first fiber layer 21 may be made through entangling its ingredient fibers with one another by use of needles, air or the like instead of by use of the high-pressure water jetting treatment.

Unlike the first fiber layer 21, the second fiber layer 22 is formed by integrally entangling its ingredient pulp fiber 24 and chemical fiber 25 through a joint formation process, instead of through the high-pressure water jetting treatment.

For the purpose of manufacturing the second web 41, types of second web former 40 can be changed depending on the intended use of the two-layered laminate wrapping sheet 70 whenever deemed necessary. It is desirable that the second web former 40 used for manufacturing the second web 41 may be, for example, of a cylinder net type or a former type which makes it possible to obtain a formation homogeneous.

A first treated web 33 obtained by causing the first high-pressure water jetting treatment unit 32 to apply the high-pressure water jetting treatment to the first web 31 is conveyed to the second web former 40. Thereafter, by a first joint former 42, the first treated web 33 thus conveyed is put on the second web 41 formed by the second web former 40, and a wet two-layered laminate wrapping sheet 43 is formed and sent out. This wet two-layered laminate wrapping sheet 43 is conveyed to a dryer 60. After dried, the two-layered laminate wrapping sheet 43 is wound up by a winder 90.

(Method of Manufacturing a Three-layered Laminated Wrapping Sheet 80)

A method of manufacturing a three-layered laminate wrapping sheet 80 is the same as the method of manufacturing a two-layered laminate wrapping sheet 70 except that the method of manufacturing a three-layered laminate wrapping sheet 80 includes a step of laminating a third fiber layer 23 onto the second fiber layer 22, the third fiber layer 23 being formed with the same method as is used to form the first fiber layer 21.

A material liquid dispersed in water with a predetermined concentration is sent to the first web former 30, the second web former 40 and a third web former 50, and the material liquid thus sent is made into the first web 31, the second web 41 and a third web 51.

A first treated web 33 obtained by causing the first high-pressure water jetting treatment unit 32 to apply a high-pressure water jetting treatment to the first web 31 is conveyed to the second web former 40. Thereafter, by the first joint former 42, the first treated web 33 thus conveyed is put on the second web 41 formed by the second web former 40.

A third treated web 53 obtained by causing a second high-pressure water jetting unit 52 to apply a high-pressure water jetting treatment to the third web 51 is conveyed. The third treated web 53 thus conveyed is put on the second web by a second joint former 54, and a wet three-layered laminate wrapping sheet 55 is formed. The wet three-layered laminate wrapping sheet 55 thus formed is conveyed to the drier 60. After dried, the three-layered laminate wrapping sheet 55 is wound up by the winder 90.

Enbodiment

Descriptions will be provided hereinbelow for the examples of the present invention. However, these examples are presented just for the purpose of describing the preferred embodiments of the present invention, and accordingly impose no restriction on the present invention at all.

For the purpose of evaluating the hydrolytic property and the like of the wrapping sheet according to the present invention, wrapping sheets were formed, and the properties thereof were checked.

[Forming of Two-layered Laminate Wrapping Sheet]

(Forming of First Fiber Layer)

A mixture of NBKP (softwood chemical pulp) (with a CSF of 720 cc) and NBKP (with a CSF of 600 cc) were used as the pulp fiber 24 of the first fiber layer. CSF stands for Canadian Standard Freeness.

Rayon fiber (trade name: Corona, manufactured by Daiwabo Rayon Co., Ltd.) with a fineness of 1.1 dtex and with an average fiber length of 7 mm was used as the chemical fiber 25 of the first fiber layer.

The pulp fiber 24 content of the first fiber layer was 50% by mass (for detail, the NBKP (with a CFS of 720 cc) content of the first fiber layer was 30% by mass, and the NBKP (with a CFS of 600 cc) content of the first fiber layer was 20% by mass). On the other hand, the chemical fiber 25 content of the first fiber layer was 50% by mass.

By use of a rectangular heater manufactured by Kumagai Riki Kogyo Co., Ltd., the pulp fiber 24 and the chemical fiber 25 were formed on a wire (trade name: LL-70E (double-woven)) manufactured by Nippon Filcon Co., Ltd. through a wet forming method. Thereby, a first web 31 was obtained. Subsequently, the ingredient fibers were entangled to each other by applying a high-pressure water jetting treatment to the two sides of the first web 31 with an energy set at 0.38 $kW/m^2$ for four times. Thereafter, the first web 31 was dried by use of a rotary drier at a temperature of 120°C for 3 minutes. By this, a first treated web 33 with a basis weight of 16.2 $g/m^2$ was made. Incidentally, the pore size of the nozzle was 95 $\mu m$, and the pores were arrayed at intervals of 0.5 mm in the cross direction CD.

(Forming of Second Fiber Layer)

A material slurry obtained by blending NBKP (with a CSF of 600 cc) and LBKP (hardwood chemical pulp) together was used for the pulp fiber 24 of the second fiber layer. The proportion of the NBKP (with a CSF of 600 cc) in the blended material slurry was 50% whereas the proportion of the LBKP in the blended material slurry was 50%. Thereby, a second web 41 was formed through a cylinder paper forming process in a way that the dry basis weight of the second web 41 was 20.4 $g/m^2$. Incidentally, the chemical fiber 25 content of the second fiber layer was 0% by mass.

The transferred first treated web 33 and the second web 41 were pressed together, and were thus integrated into a wet two-layered laminate wrapping sheet. After drying through dehydration, a two-layered laminate wrapping sheet 70 with a basis weight of 36.8 $g/m^2$ was obtained. The wrapping sheet thus obtained is referred to as "example 1."

[Measurement of Dry Strength]

By use of a test instrument Tensilon (trade name; manufactured by A&D Co., Ltd.), the dry strength of example 1 was measured with a condition that the width of the sample piece was 25 mm; the length thereof was 150 mm; the chuck interval thereof was 100 mm; and the tensile speed thereof was 100 mm/min. The dry strength of example 1 was measured both in the machine direction MD and the cross direction CD. Incidentally, the dry strength thereof means the maximum dry tensile strength of thereof.

[Measurement of Wet strength]

Example 1 was left to stand still in a closed top container with ion-exchanged water being impregnated into example 1 with a moisture content of example 1 (or the weight ratio of moisture to the base fabric) being 300% for three hours. Thereafter, by use of the test instrument Tensilon (trade name; manufactured by A&D Co., Ltd.), the wet strength of example 1 was measured with a condition that the width of the sample piece was 25 mm; the length thereof was 150 mm; the chuck interval thereof was 100 mm; and the tensile speed thereof was 100 mm/min. The wet strength of example 1 was measured both in the machine direction MD and the cross direction CD. Incidentally, the wet strength thereof means the maximum wet tensile strength thereof.

[Measurement of Dust Permeation]

3 mg of test dust was placed on example 1, which was placed on a piece of filter paper, for each of the three particle sizes of the test dust. Subsequently, each 3 mg of test dust was suctioned by an aspirator from under the filter paper for 30 seconds. Thereafter, how much of the test dust was adhered to the piece of filter paper after permeating example 1 was visually evaluated for each particle size of the test dust. A result of the visual evaluation is shown in Table 1: a case no test dust adhered to the filter paper is indicated by a circle; a case where a minute amount of test dust permeated the example to adhere to the filter paper but did not fall even when the filter paper was turned upside down is indicated by a triangle; and a case where some test dust adhered to the filter paper and fell when the filter paper was turned upside down is indicated by a cross.

[Measurement of Air-flow Resistance]

The air-flow resistance of example 1 was measured with a measuring instrument (trade name: KES-F8, manufactured by Kato Tech Co., Ltd.) with the cylinder-side part of example 1 being put downward. An average of five measurements of the air-flow resistance of example 1 is shown in Table 1.

[Fluff Test and Measurement]

How much example 1 fluffed was measured by adhering a mold-release-side surface of a piece of fabric tape to the friction surface of a rubbing tester for color fastness (manufactured by Daiei Kagaku Seiki MFG. Co., Ltd.) on example 1, the sample piece having a width of 300 mm and a length of 200 mm. The piece of fabric tape was reciprocated for five times while applying a load of 200 g to example 1: A result of the fluffing test is shown in Table 1: a case where an example did not fluff is indicated by a circle; a case where an example partially fluffed a little, but no ingredient fiber peeled off, is indicated by a triangle; and a case where an example fluffed overall, and/or ingredient fibers peeled off, is indicated by a cross.

[Measurement of Hydrolytic Property by Use of Shake Flask Method]

First of all, 800 cc of distilled water was poured into a 1000-ml flask. Subsequently, a squared piece of example 1 with a size of 10 cmx10 cm was placed in the distilled water. Thereafter, the squared piece of example in the distilled water was shaken with a shaker (trade name: SHKV-200, manufactured by IWAKI Co. Ltd.) at a shaking speed of 240 rpm for 30 minutes. A result of measuring the hydrolytic property thereof is shown in Table 1: a case where an example was so dispersed in the distilled water that no trace of its original form remained or even the largest remaining piece of the example became 50 $cm^2$ or less in size are indicated by a circle; a case where an example was so dispersed in the distilled water that the largest remaining piece of the example was still more than 50 $cm^2$ in size is indicated by a triangle; and a case where an example was not dispersed in the distilled water so that the original form of the example remained is indicated by a cross.

In addition to the wrapping sheet according to example 1, wrapping sheets according to examples 2 to 6 were made by adding a dye and a sizing agent to each wrapping sheet to such an extent that the hydrolytic property of each wrapping sheet was maintained, and by changing the pulp fiber 24 content, the chemical fiber 25 content, the basis weight and the like from one wrapping to another. Subsequently, the properties of each of the wrapping sheets according to examples 2 to 6 were tested or measured in the same manner as the properties of the wrapping sheet according to example 1. Incidentally, the wrapping sheets according to examples 2 to 6 were the same size as the wrapping sheet according to example 1, and were shaped the same as the wrapping sheet according to example 1.

[Forming of Three-layered Wrapping Sheet]

[Forming of First Fiber Layer]

NBKP (with a CSF of 720 cc) and a synthetic pulp (trade name: SWP E-400, manufactured by Mitsui Chemicals, Inc.) were mixedly used as the pulp fiber 24 of the first fiber layer.

Rayon fiber (trade name: Corona, manufactured by Daiwabo Rayon Co., Ltd.) with a fineness of 1.1 dtex and with an average fiber length of 7 mm was used as the chemical fiber 25 of the first fiber layer.

The pulp fiber 24 content of the first fiber layer was 60% by mass (for detail, the NBKP (with a CFS of 750 cc) content of the first fiber layer was 50% by mass, and the synthetic pulp content of the first fiber layer was 10% by mass, where the synthetic pulp was SWP E-400 (trade name) manufactured by Mitsui Chemicals, Inc.). On the other hand, the chemical fiber 25 content of the first fiber layer was 40% by mass.

By use of the rectangular heater manufactured by Kumagai Riki Kogyo Co., Ltd., the pulp fiber 24 and the chemical fiber 25 were formed on the wire (trade name: LL-70E (double-woven)) manufactured by Nippon Filcon Co., Ltd. through a wet forming process. Thereby, a first web 31 was obtained. Subsequently, the ingredient fibers were entangled to each other by applying a high-pressure water jetting treatment to the two sides of the first web 31 with an energy set at 0.38 kW/m$^2$ four times. Thereafter, the first web 31 was dried by use of the rotary drier at a temperature of 120°C for 3 minutes. By this, a first treated web 33 with a basis weight of 16.4 g/m$^2$ was made. Incidentally, the pore size of the nozzle was 95 μm, and the pores were arrayed at intervals of 0.5 mm in the cross direction CD.

(Forming of Second Fiber Layer)

A material slurry obtained by blending NBKP (with a CSF of 600 cc) and LBKP together was used for the pulp fiber 24 of the second fiber layer 22. The proportion of the NBKP (with a CSF of 600 cc) in the blended material slurry was 50% whereas the proportion of the LBKP in the blended material slurry was 50%. Thereby, a second web 41 was formed through a cylinder paper forming process in a way that the dry basis weight of the second web 41 was 20.4 g/m$^2$. Incidentally, the chemical fiber 25 content of the second fiber layer was 0% by mass.

(Forming of Third Fiber Layer)

NBKP (with a CSF of 720 cc) and a synthetic pulp (trade name: SWP E-400, manufactured by Mitsui Chemicals, Inc.) were mixedly used as the pulp fiber 24 of the third fiber layer 23.

Rayon fiber (trade name: Corona, manufactured by Daiwabo Rayon Co., Ltd.) with a fineness of 1.1 dtex and with an average fiber length of 7 mm was used as the chemical fiber 25 of the third fiber layer.

The pulp fiber 24 content of the third fiber layer 23 was 60% by mass (for detail, the NBKP (with a CFS of 750 cc) content of the first third layer was 50% by mass, and the synthetic pulp (trade name: SWP E-400, manufactured by Mitsui Chemicals, Inc) content of the first fiber layer was 10% by mass). On the other hand, the chemical fiber 25 content of the first fiber layer was 40% by mass.

By use of the rectangular heater manufactured by Kumagai Riki Kogyo Co., Ltd., the pulp fiber 24 and the chemical fiber 25 were formed on the wire (trade name: LL-70E (double-woven)) manufactured by Nippon Filcon Co., Ltd. through a wet forming process. Thereby, a third web 51 was obtained. Subsequently, the ingredient fibers were entangled to each other by applying a high-pressure water jetting treatment to the two sides of the third web 51 with an energy set at 0.38 kW/m$^2$ four times. Thereafter, the third web 51 was dried by use of the rotary drier at a temperature of 120°C for 3 minutes. By this, a third treated web 53 with a basis weight of 16.4 g/m$^2$ was made. Incidentally, the pore size of the nozzle was 95 μm, and the pores were arrayed at intervals of 0.5 mm in the cross direction CD.

The transferred first treated web 33 was put on the second web 41, and the third processed web 53 is subsequently put on the resultant second web 41. Thereby, the first treated web 33, the second web 41 and the third processed web 53 are pressed together with the three webs being stacked one on another, and were thus integrated into a wet three-layered laminate wrapping sheet. After drying through dehydration, a three-layered laminate wrapping sheet 80 with a basis weight of 53.2 g/m$^2$ was obtained. The wrapping sheet thus obtained is referred to as "example 7."

Example 7 was measured in the same manner as example 1 was measured in terms of the dry strength, the wet strength, the dust permeation, the air-flow resistance, the fluff test and hydrolytic property by the shake flask method. Incidentally, example 7 was the same size and shape as example 1.

Wrapping sheets according to comparative examples 1 to 5 were made as follows, and were subsequently tested and measured in the same manner as the wrapping sheet according to example 1. Incidentally, the wrapping sheets according to comparative examples 1 to 5 were the same size and shape as the wrapping sheet according to example 1.

[Comparative Example 1]

Comparative example 1 was made in the same manner as example 1 was, except that the basis weight of the first fiber layer 21 was 21.4 g/m$^2$ whereas the basis weight of the second fiber layer 22 was 10 g/m$^2$.

[Comparative Example 2]

Comparative example 2 was made in the same manner as example 1 was, except that the first fiber layer 21 was made by applying no high-pressure water jetting treatment, and except that the basis weight of the second fiber layer 22 was 20 g/m$^2$.

[Comparative Example 3]

Comparative example 3 was made in the same manner as example 1 was, except that the basis weight of the first fiber layer 21 was 32.5 g/m$^2$ whereas the basis weight of the second fiber layer 22 was 31.3 g/m$^2$.

[Comparative Example 4]

Comparative Example 4 was made in the same manner as example 1 was made, except that comparative example 4 was a single-layered wrapping sheet made as follows. For the pulp fiber 24 of comparative example 4, a fiber material was made by blending NBKP (with a CSF of 720 cc) and rayon fiber (trade name: Corona, manufactured by Daiwabo Rayon Co., Ltd.) with a fineness of 1.1 dtex and with an average fiber length of 7 mm. The proportion of the NBKP in the blended fiber material was 50% by mass, and the proportion of the rayon fiber in the blended fiber material was 50% by mass. A cationic dye (CI. Direct Red 81, trade name: Sumilight Red 4B, manufactured by Taoka Chemical Co., Ltd.) was added to the fiber material and was agitated therein when the NBKP and the rayon fiber were blended together. Subsequently, a cationic sizing agent (or a cationic styrene resin) (trade name: Size Pine W-360, manufactured by Arakawa Chemical Industries, Ltd.) was added to the resultant fiber material and was agitated therein. By using the resultant fiber material as a material slurry, a first treated web 33 was made with the same method as the first treated web 33 according to example 1, and was subsequently dried directly. Thus, the single-layered wrapping sheet which had a basis weight of 30 g/m$^2$, and which consisted of the first fiber layer 21 only, was made.

[Comparative Example 5]

Comparative Example 5 was made as the same manner as example 1 was made, except that comparative example 5 was a single-layered wrapping sheet made as flows . For the pulp fiber 24 of comparative example 5, a fiber material was made by blending NBKP (with a CSF of 600 cc) and LBKP together. The proportion of the NBKP in the blended fiber material was 50% by mass, and the proportion of the LBKP in the blended fiber material was 50% by mass. A second web 41 was made of the fiber material with the same method as the second web 41 according to example 1, and thereafter was dried directly. Thereby, the single-layered wrapping sheet which had a basis weight of 20.4 g/m$^2$, and which consisted of the second fiber layer 22 only, was made.

# TABLE. 1

| | | EXAMPLE 1 | | EXAMPLE 2 | | EXAMPLE 3 | | EXAMPLE 4 | | EXAMPLE 5 | | EXAMPLE 6 | | EXAMPLE 7 | | | COMPARATIVE EXAMPLE 1 | | COMPARATIVE EXAMPLE 2 | | COMPARATIVE EXAMPLE 3 | | COMPARATIVE EXAMPLE 4 | COMPARATIVE EXAMPLE 5 |
|---|---|---|---|---|---|---|---|---|---|---|---|---|---|---|---|---|---|---|---|---|---|---|---|---|---|
| | | FIRST LAYER (SL) | SECOND LAYER (CYLINDER) | FIRST LAYER (SL) | SECOND LAYER (CYLINDER) | FIRST LAYER (SL) | SECOND LAYER (CYLINDER) | FIRST LAYER (SL) | SECOND LAYER (CYLINDER) | FIRST LAYER (SL) | SECOND LAYER (CYLINDER) | FIRST LAYER (SL) | SECOND LAYER (CYLINDER) | FIRST LAYER (SL) | SECOND LAYER (CYLINDER) | THIRD LAYER (SL) | FIRST LAYER (SL) | SECOND LAYER (CYLINDER) | FIRST LAYER (INCLINED) | SECOND LAYER (CYLINDER) | FIRST LAYER (SL) | SECOND LAYER (CYLINDER) | SINGLE LAYER (SL) | SINGLE LAYER (SL) |
| NBKP | csf720cc | 30% | | 50% | | 50% | | 50% | | 50% | | 70% | | 50% | | 50% | 30% | | 50% | | 30% | | 50% | |
| NBKP | csf600cc | 20% | 50% | | 40% | | 40% | | 40% | | 45% | | 35% | | 50% | | 20% | 50% | | 40% | 20% | 50% | | 50% |
| LBKP | FREE | | 50% | | 40% | | 40% | | 40% | | 45% | | 35% | | 50% | | | 50% | | 40% | | 50% | | 50% |
| RAYON | 1.1dt*7mm | 50% | | 40% | | 40% | | 40% | | 50% | | 30% | | 40% | | 40% | 50% | | 40% | | 50% | | 50% | |
| SYNTHETIC PULP | SWP E-400 | | | 10% | 20% | 10% | 20% | | 20% | | 10% | | 30% | 10% | | 10% | | | 10% | 20% | | | | |
| SPLITTABLE FIBER | 3.3dt*5mm | | | | | | | 10% | | | | | | | | | | | | | | | | | |
| CATIONIC DYE | sunlight Red 4B | | | 0.024% | | 0.024% | | 0.024% | | 0.024% | | 0.024% | | 0.024% | | | | | 0.024% | | | | 0.024% | 0.024% |
| CATIONIC DYE | kayacel turquoise | | | | | | | | | | | | | | 0.22% | | | | | | | | | |
| SIZING AGENT | SIZE PINE W-360 | | | 3% | 3% | 3% | 3% | 3% | 3% | 3% | 3% | 3% | 3% | 3% | 3% | | | | 3% | 3% | | | 3% | 3% |
| BASIS WEIGHT OF EACH LAYER | g/m² | 16.2 | 20.4 | 16.1 | 15.2 | 16.4 | 19.1 | 16.5 | 15.2 | 14.8 | 20.0 | 20.7 | 19.8 | 16.4 | 20.4 | 16.4 | 21.4 | 10.0 | 16.1 | 20.0 | 32.5 | 31.3 | 42.2 | 30.0 |
| TOTAL BASIS WEIGHT | g/m² | 36.8 | | 31.3 | | 35.5 | | 31.7 | | 34.8 | | 40.5 | | 53.2 | | | 31.4 | | 36.1 | | 63.8 | | 42.2 | 30.0 |
| THICKNESS | mm | 0.12 | | 0.09 | | 0.10 | | 0.09 | | 0.10 | | 0.11 | | 0.18 | | | 0.12 | | 0.10 | | 0.20 | | 0.15 | 0.06 |
| DENSITY | g/cm³ | 0.307 | | 0.348 | | 0.355 | | 0.352 | | 0.348 | | 0.368 | | 0.296 | | | 0.260 | | 0.361 | | 0.319 | | 0.287 | 0.517 |
| DRY STRENGTH N/25mm | MD | 18.91 | | 25.35 | | 29.89 | | 26.27 | | 34.58 | | 40.68 | | 51.32 | | | 16.21 | | 13.33 | | 54.10 | | 7.71 | 41.50 |
| | CD | 7.95 | | 8.75 | | 10.22 | | 8.16 | | 14.05 | | 16.60 | | 17.55 | | | 5.11 | | 4.27 | | 19.20 | | 7.02 | 13.00 |
| WET STRENGTH N/25mm | MD | 2.67 | | 5.04 | | 6.00 | | 2.19 | | 2.47 | | 2.26 | | 8.80 | | | 2.09 | | 1.59 | | 10.17 | | 2.85 | 0.46 |
| | CD | 1.27 | | 1.51 | | 1.89 | | 1.78 | | 1.29 | | 1.11 | | 2.93 | | | 1.04 | | 0.66 | | 4.15 | | 2.82 | 0.14 |
| DUST PERMEATION | 75μm-1mm | O | | O | | O | | O | | O | | O | | O | | | O | | O | | O | | X | O |
| | 15-20μm | O | | O | | O | | O | | O | | O | | O | | | △ | | O | | O | | X | O |
| | 5μm | O | | O | | O | | O | | O | | O | | O | | | X | | O | | O | | X | O |
| AIR-FLOW RESISTANCE VALUE | Kpa·s/m | 3.07 | | 6.36 | | 7.25 | | 7.01 | | 6.91 | | 9.23 | | 5.37 | | | 2.95 | | 7.63 | | 10.27 | | 0.212 | 13.79 |
| FLUFF | | O | | O | | O | | O | | O | | O | | O | | | △ | | X | | O | | O | O |
| HYDROLYTIC PROPERTY | SHAKE FLASK METHOD | O | | O | | O | | O | | O | | O | | △ | | | O | | O | | X | | O | O |

EP 2 163 378 B1

It is learned from Table 1 that examples 1 to 7 exhibited a good dust permeation, dry strength, wet strength, hydrolytic property. Accordingly, while examples 1 to 7 exhibit those properties, they have a certain degree of strength.

By contrast, some of examples 1 to 5 exhibited an enhanced hydrolytic property while exhibiting a degraded dry strength, wet strength and the like. The others of examples 1 to 5 exhibited an enhanced dry strength and wet strength while exhibiting a degraded hydrolytic property. Yet some of examples 1 to 5 exhibited an enhanced hermetic property while exhibiting a degraded hydrolytic property and lint-free property. Based on this fact, it is learned that, unlike the present invention, comparative examples 1 to 5 was not capable of enhancing the hermetic property and hydrolytic property while having a certain degree of strength.

INDUSTRIAL APPLICABILITY

As described above, individual package of absorbent article, wrapping sheet, and method of manufacturing wrapping sheet according to the present invention provides an individual package and a wrapping sheet which both exhibit a hydrolytic property, a soft tactile impression and appearance, and a high air tightness property which does not allow foreign matters to go in beyond the individual package and the wrapping sheet. In addition, the present invention employs a wet paper-forming and layer-combining process with a paper machine to manufacture wrapping sheets with a laminated structure. Thus, it makes a secondary process unnecessary, and it achieves cost reduction. Therefore, the present invention is useful.

**Claims**

1. A wrapping sheet for an absorbent article including at least an absorbent exhibiting a fluid retention property, comprising:

a first fiber layer formed by application of a high-pressure water jetting treatment, the first fiber layer including pulp fiber and chemical fiber, a pulp fiber content of the first fiber layer being 30% by mass to 70% by mass, a chemical fiber content of the first fiber layer being 70% by mass and 30% by mass, an average fiber length of the chemical fiber being not more than 20 mm; and

a second fiber layer arranged by laminating the second fiber layer to the first fiber layer, the second fiber layer including pulp fiber, a pulp fiber content of the second fiber layer being 70% by mass to 100% by mass; **characterised in that** the second fiber layer includes chemical fiber not more than 30% by mass with an average fiber length of 20 mm or less and with a fiber diameter smaller than that of the pulp fiber.

2. The wrapping sheet according to claim 1, further comprising a third fiber layer formed by application of a high-pressure water jetting treatment and arranged by laminating the third fiber layer to one side of the second fiber layer with the first fiber layer laminated to the other side of the second fiber layer, the third fiber layer including pulp fiber and chemical fiber, a pulp fiber content of the third fiber layer being 30% by mass to 70% by mass, a chemical fiber content of the third fiber layer being 70% by mass to 30% by mass, an average fiber length of the chemical fiber being not more than 20 mm.

3. The wrapping sheet according to claim 1 or claim 2, wherein when a 10 cmx10 cm sample piece of wrapping sheet placed in 800 cc of distilled water is shaken at a shaking speed of 240 rpm for 30 minutes, the sample piece is so dispersed in the distilled water that the largest remaining piece of wrapping sheet is not more than 50 cm$^2$ in size.

4. The wrapping sheet according to any one of claims 1 to 3, wherein
a basis weight of the first fiber layer is 10 g/m$^2$ to 30 g/m$^2$,
a basis weight of the second fiber layer is 15 g/m$^2$ to 30 g/m$^2$, and
a basis weight of the wrapping sheet is 25 g/m$^2$ to 60 g/m$^2$.

5. The wrapping sheet according to any one of claims 1 and 3, wherein
a basis weight of the first fiber layer is 10 g/ m$^2$ to 30 g/ m$^2$,
a basis weight of the second fiber layer is 15 g/ m$^2$ to 30 g/ m$^2$,
a basis weight of the third fiber layer is 10 g/ m$^2$ to 30 g/ m$^2$, and
a basis weight of the wrapping sheet is 35 g/ m$^2$ to 60 g/ m$^2$.

6. The wrapping sheet according to any one of claims 1 to 5, wherein
a pattern is formed in at least one of the first fiber layer and the second fiber layer by changing the basis weight of the first fiber layer and/or the second fiber.

7. The wrapping sheet according to any one of claims 1 to 6, wherein
at least one of the first fiber layer and the second fiber layer is colored.

8. The wrapping sheet according to claim 7, wherein
a color in which the first fiber layer is colored is different from color in which the second fiber layer is colored.

9. The wrapping sheet according to any one of claims 1 to 8, wherein
at least one of the first fiber layer to the three fiber layer includes a sizing agent, and at least partially exhibits a predetermined water resisting property.

10. An individual package of an absorbent article, comprising:

an absorbent article at least including an absorbent exhibiting a fluid retention property; and
a wrapping material wrapping the absorbent article, the wrapping material constituted of the wrapping sheet according to any one of claims 1 to 9.

11. A method of manufacturing a wrapping sheet, comprising the steps of:

forming a first fiber layer from pulp fiber and chemical fiber into a fiber web in a wet paper-forming method, and subsequently by applying a high-pressure water jetting treatment to at least one side of the fiber web to entangle the pulp fiber and the chemical fiber with each other a pulp fiber content of the first fiber layer being 30% by mass to 70% by mass, a chemical fiber content of the first fiber layer being 70% by mass and 30% by mass, an average fiber length of the chemical fiber being not more than 20 mm;
forming a second fiber layer from pulp fiber and chemical fiber the second fiber layer including pulp fiber, a pulp fiber content of the second fiber layer being 70% by mass to 100% by mass;
**characterised by** the step of including into the second fiber layer chemical fiber not more than 30% by mass with an average fiber length of 20 mm or less and with a fiber diameter smaller than that of the pulp fiber; and
conveying the first fiber layer, subsequently integrally laminating the first fiber layer and the second fiber layer to each other while the second fiber layer remains wet, and thereafter drying the laminated first and second fiber layers

12. The method of manufacturing a wrapping sheet according to claim 11, further comprising the steps of:

forming a third fiber layer from pulp fiber and chemical fiber into a fiber web in a wet paper-forming method, and subsequently by applying a high-pressure water jetting treatment to at least one side of the fiber web to entangle the pulp fiber and the chemical fiber with each other; and
conveying the first and second fiber layers after integrally laminating the first fiber layer and the second fiber layer to each other while the first and second fiber layer remain wet, subsequently integrally laminating the third fiber layer onto the first and second fiber layers while the third fiber layer remains wet, and thereafter drying the laminated first to third fiber layers.

13. The method of manufacturing a wrapping sheet according to any one of claims 11 and 12, wherein
in at least one of the step of forming the first fiber layer and the step of forming the third fiber layer, the fiber web is transferred from a fiber layer forming wire to a patterning wire after the fiber web is formed, and subsequently the high-pressure water jetting treatment is applied to the fiber web thus transferred.

14. The method of manufacturing a wrapping sheet according to any one of claims 11 to 13,
wherein, for at least one of the first and third fiber layers, the fiber web is formed by a paper-forming method of any one of a tanmo type and a fourdrinier type.

15. The method of manufacturing a wrapping sheet according to any one of claims 11, 12 and 14, wherein the high-pressure water jetting treatment is applied to the fiber web placed on the fiber layer forming wire.

16. The method of manufacturing a wrapping sheet according to any one of claims 11 to 15, wherein, for the second fiber layer, the fiber web is formed by a paper-forming method of any one of a cylinder type and a former type.

**Patentansprüche**

1. Ein Verpackungsmaterialbogen für einen saugfähigen Artikel einschließlich mindestens einem saugfähigen Mittel, das eine Flüssigkeitsretentionseigenschaft aufweist, beinhaltend:

   eine erste Faserschicht, die durch Anwendung einer Hochdruck-Wasserstrahlbehandlung gebildet wird, wobei die erste Faserschicht Zellstofffaser und chemische Faser umfasst, wobei ein Zellstofffasergehalt der ersten Faserschicht 30 Massenprozent bis 70 Massenprozent beträgt, ein Gehalt der chemischen Faser der ersten Faserschicht 70 Massenprozent und 30 Massenprozent beträgt, eine durchschnittliche Faserlänge der chemischen Faser nicht mehr als 20 mm beträgt; und
   eine zweite Faserschicht durch das Laminieren der zweiten Faserschicht an die erste Faserschicht angeordnet ist, wobei die zweite Faserschicht Zellstofffaser umfasst, wobei ein Zellstofffasergehalt der zweiten Faserschicht 70 Massenprozent bis 100 Massenprozent beträgt; **dadurch gekennzeichnet, dass** die zweite Faserschicht chemische Faser zu nicht mehr als 30 Massenprozent mit einer durchschnittlichen Faserlänge von 20 mm oder weniger und mit einem Faserdurchmesser, der kleiner als der der Zellstofffaser ist, umfasst.

2. Verpackungsmaterialbogen gemäß Anspruch 1, das ferner eine dritte Faserschicht beinhaltet, die durch Anwendung einer Hochdruck-Wasserstrahlbehandlung gebildet wird und durch Laminieren der dritten Faserschicht an eine Seite der zweiten Faserschicht angeordnet wird, wobei die erste Faserschicht an die andere Seite der zweiten Faserschicht laminiert wird, wobei die dritte Faserschicht Zellstofffaser und chemische Faser umfasst, wobei ein Zellstofffasergehalt der dritten Faserschicht 30 Massenprozent bis 70 Massenprozent beträgt, ein Gehalt der chemischen Faser der dritten Faserschicht 70 Massenprozent bis 30 Massenprozent beträgt, eine durchschnittliche Faserlänge der chemischen Faser nicht mehr als 20 mm beträgt.

3. Verpackungsmaterialbogen gemäß Anspruch 1 oder Anspruch 2, wobei, wenn ein Probestück des Verpackungsmaterialbogens von 10 cm x 10 cm in 800 cm$^3$ destilliertem Wasser bei einer Schüttelgeschwindigkeit von 240 rpm 30 Minuten lang geschüttelt wird, das Probestück so in dem destillierten Wasser dispergiert wird, dass das größte verbleibende Stück des Verpackungsmaterialbogens nicht größer als 50 cm$^2$ ist.

4. Verpackungsmaterialbogen gemäß einem der Ansprüche 1 bis 3, wobei ein Basisgewicht der ersten Faserschicht 10 g/m$^2$ bis 30 g/m$^2$ beträgt,
   ein Basisgewicht der zweiten Faserschicht 15 g/m$^2$ bis 30 g/m$^2$ beträgt, und
   ein Basisgewicht des Verpackungsmaterialbogens 25 g/m$^2$ bis 60 g/m$^2$ beträgt.

5. Verpackungsmaterialbogen gemäß einem der Ansprüche 1 und 3, wobei
   ein Basisgewicht der ersten Faserschicht 10 g/m$^2$ bis 30 g/m$^2$ beträgt,
   ein Basisgewicht der zweiten Faserschicht 15 g/m$^2$ bis 30 g/m$^2$ beträgt,
   ein Basisgewicht der dritten Faserschicht 10 g/m$^2$ bis 30 g/m$^2$ beträgt, und
   ein Basisgewicht des Verpackungsmaterialbogens 35 g/m$^2$ bis 60 g/m$^2$ beträgt.

6. Verpackungsmaterialbogen gemäß einem der Ansprüche 1 bis 5, wobei durch das Ändern des Basisgewichts der ersten Faserschicht und/oder der zweiten Faser ein Muster in mindestens einem der ersten Faserschicht und der zweiten Faserschicht gebildet wird.

7. Verpackungsmaterialbogen gemäß einem der Ansprüche 1 bis 6, wobei mindestens eine der ersten Faserschicht und der zweiten Faserschicht gefärbt ist.

8. Verpackungsmaterialbogen gemäß Anspruch 7, wobei
   sich eine Farbe, in der die erste Faserschicht gefärbt ist, von einer Farbe, in der die zweite Faserschicht gefärbt ist, unterscheidet.

9. Verpackungsmaterialbogen gemäß einem der Ansprüche 1 bis 8, wobei mindestens eine der ersten Faserschicht zu der drei Faserschicht ein Leimungsmittel umfasst, und mindestens teilweise eine vorbestimmte Wasser widerstehende Eigenschaft aufweist.

10. Eine Einzelverpackung eines saugfähigen Artikels, beinhaltend:

    einen saugfähigen Artikel, der mindestens ein saugfähiges Mittel, das eine Flüssigkeitsretentionseigenschaft aufweist, umfasst; und

ein Verpackungsmaterial, das den saugfähigen Artikel umhüllt, wobei das Verpackungsmaterial aus dem Verpackungsmaterialbogen gemäß einem der Ansprüche 1 bis 9 besteht.

**11.** Ein Verfahren zur Herstellung eines Verpackungsmaterialbogens, das die folgenden Schritte beinhaltet:

Bilden einer ersten Faserschicht aus Zellstofffaser und chemischer Faser in eine Faserbahn in einem Nasspapierbildungsverfahren, und nachfolgend durch Anwenden einer Hochdruck-Wasserstrahlbehandlung auf mindestens eine Seite der Faserbahn, um die Zellstofffaser und die chemische Faser miteinander zu verwickeln, wobei ein Zellstofffasergehalt der ersten Faserschicht 30 Massenprozent bis 70 Massenprozent beträgt, wobei ein Gehalt der chemischen Faser der ersten Faserschicht 70 Massenprozent und 30 Massenprozent beträgt, wobei eine durchschnittliche Faserlänge der chemischen Faser 20 mm nicht übersteigt;
Bilden einer zweiten Faserschicht aus Zellstofffaser und chemischer Faser, wobei die zweite Faserschicht Zellstofffaser umfasst, wobei ein Zellstofffasergehalt der zweiten Faserschicht 70 Massenprozent bis 100 Massenprozent beträgt;
**gekennzeichnet durch** den Schritt des Umfassens von nicht mehr als 30 Massenprozent chemischer Faser in der zweiten Faserschicht mit einer durchschnittlichen Faserlänge von 20 mm oder weniger und mit einem Faserdurchmesser, der kleiner als der der Zellstofffaser ist; und
Fördern der ersten Faserschicht, nachfolgend ganzheitliches Laminieren der ersten Faserschicht und der zweiten Faserschicht aneinander, während die zweite Faserschicht nass bleibt, und danach Trocknen der laminierten ersten und zweiten Faserschichten.

**12.** Verfahren zur Herstellung eines Verpackungsmaterialbogens gemäß Anspruch 11, das ferner die folgenden Schritte beinhaltet:

Bilden einer dritten Faserschicht aus Zellstofffaser und chemischer Faser in eine Faserbahn in einem Nasspapierbildungsverfahren, und nachfolgendes Anwenden einer Hochdruck-Wasserstrahlbehandlung auf mindestens eine Seite der Faserbahn, um die Zellstofffaser und die chemische Faser miteinander zu verwickeln; und
Fördern der ersten und zweiten Faserschichten nach dem ganzheitlichen Laminieren der ersten Faserschicht und der zweiten Faserschicht aneinander, während die erste und zweite Faserschicht nass bleiben, nachfolgend ganzheitliches Laminieren der dritten Faserschicht an die erste und zweite Faserschichten, während die dritte Faserschicht nass bleibt, und danach Trocknen der laminierten ersten bis dritten Faserschichten.

**13.** Verfahren zur Herstellung eines Verpackungsmaterialbogens gemäß einem der Ansprüche 11 und 12, wobei in mindestens einem des Schritts des Bildens der ersten Faserschicht und des Schritts des Bildens der dritten Faserschicht die Faserbahn von einem faserschichtbildenden Draht zu einem Musterungsdraht übertragen wird, nachdem die Faserbahn gebildet ist, und nachfolgend die Hochdruck-Wasserstrahlbehandlung auf die so übertragene Faserbahn angewendet wird.

**14.** Verfahren zur Herstellung eines Verpackungsmaterialbogens gemäß einem der Ansprüche 11 bis 13, wobei für mindestens eine der ersten und dritten Faserschichten die Faserbahn durch ein papierbildendes Verfahren gemäß einem von einem Tanmo-Typ und einem Fourdrinier-Typ gebildet wird.

**15.** Verfahren zur Herstellung eines Verpackungsmaterialbogens gemäß einem der Ansprüche 11, 12 und 14, wobei die Hochdruck-Wasserstrahlbehandlung auf die auf dem Draht zur Bildung der Faserschicht platzierte Faserbahn angewendet wird.

**16.** Verfahren zur Herstellung eines Verpackungsmaterialbogens gemäß einem der Ansprüche 11 bis 15, wobei für die zweite Faserschicht die Faserbahn durch ein papierbildendes Verfahren eines Zylindertyps und eines Formertyps gebildet wird.

**Revendications**

**1.** Une feuille d'emballage pour un article absorbant incluant au moins un absorbant présentant une propriété de rétention de fluide, comprenant :

une première couche de fibres formée par application d'un traitement à jets d'eau haute pression, la première couche de fibres incluant de la fibre de pâte à papier et de la fibre chimique, une teneur en fibre de pâte à papier de la première couche de fibres allant de 30 % en masse à 70 % en masse, une teneur en fibre chimique de la première couche de fibres étant de 70 % en masse et de 30 % en masse, une longueur de fibre moyenne de la fibre chimique ne faisant pas plus de 20 mm ; et

une deuxième couche de fibres disposée par stratification de la deuxième couche de fibres sur la première couche de fibres, la deuxième couche de fibres incluant de la fibre de pâte à papier, une teneur en fibre de pâte à papier de la deuxième couche de fibres allant de 70 % en masse à 100 % en masse ; **caractérisée en ce que** la deuxième couche de fibres inclut de la fibre chimique ne faisant pas plus de 30 % en masse avec une longueur de fibre moyenne de 20 mm ou moins et avec un diamètre de fibre plus petit que celui de la fibre de pâte à papier.

2. La feuille d'emballage selon la revendication 1, comprenant en sus une troisième couche de fibres formée par application d'un traitement à jets d'eau haute pression et disposée par stratification de la troisième couche de fibres sur un côté de la deuxième couche de fibres avec la première couche de fibres stratifiée sur l'autre côté de la deuxième couche de fibres, la troisième couche de fibres incluant de la fibre de pâte à papier et de la fibre chimique, une teneur en fibre de pâte à papier de la troisième couche de fibres allant de 30 % en masse à 70 % en masse, une teneur en fibre chimique de la troisième couche de fibres allant de 70 % en masse à 30 % en masse, une longueur de fibre moyenne de la fibre chimique ne faisant pas plus de 20 mm.

3. La feuille d'emballage selon la revendication 1 ou la revendication 2, où lorsqu'une pièce échantillon de 10 cm x 10 cm de feuille d'emballage placée dans 800 cm$^3$ d'eau distillée est agitée à une vitesse d'agitation de 240 tr/min pendant 30 minutes, la pièce échantillon est si dispersée dans l'eau distillée que la plus grande pièce restante de feuille d'emballage est d'une taille qui ne fait pas plus de 50 cm$^2$.

4. La feuille d'emballage selon l'une quelconque des revendications 1 à 3, où
un poids surfacique de la première couche de fibres va de 10 g/m$^2$ à 30 g/m$^2$,
un poids surfacique de la deuxième couche de fibres va de 15 g/m$^2$ à 30 g/m$^2$, et
un poids surfacique de la feuille d'emballage va de 25 g/m$^2$ à 60 g/m$^2$.

5. La feuille d'emballage selon l'une quelconque des revendications 1 et 3, où
un poids surfacique de la première couche de fibres va de 10 g/m$^2$ à 30 g/m$^2$,
un poids surfacique de la deuxième couche de fibres va de 15 g/m$^2$ à 30 g/m$^2$,
un poids surfacique de la troisième couche de fibres va de 10 g/m$^2$ à 30 g/m$^2$, et
un poids surfacique de la feuille d'emballage va de 35 g/m$^2$ à 60 g/m$^2$.

6. La feuille d'emballage selon l'une quelconque des revendications 1 à 5, où un motif est formé dans l'une au moins des première couche de fibres et deuxième couche de fibres par changement du poids surfacique de la première couche de fibres et/ou de la deuxième fibre.

7. La feuille d'emballage selon l'une quelconque des revendications 1 à 6, où
l'une au moins des première couche de fibres et deuxième couche de fibres est colorée.

8. La feuille d'emballage selon la revendication 7, où
une couleur dans laquelle est colorée la première couche de fibres est différente de la couleur dans laquelle est colorée la deuxième couche de fibres.

9. La feuille d'emballage selon l'une quelconque des revendications 1 à 8, où
l'une au moins de la première couche de fibres à la couche de fibres trois inclut un agent de collage, et présente au moins partiellement une propriété de résistance à l'eau prédéterminée.

10. Un conditionnement individuel d'un article absorbant, comprenant :

un article absorbant incluant au moins un absorbant présentant une propriété de rétention de fluide ; et
un matériau d'emballage emballant l'article absorbant, le matériau d'emballage étant constitué de la feuille d'emballage selon l'une quelconque des revendications 1 à 9.

11. Une méthode de fabrication d'une feuille d'emballage, comprenant les étapes consistant :

à former une première couche de fibres à partir de fibre de pâte à papier et de fibre chimique en une nappe de fibres selon une méthode de formation de papier humide, et par application ultérieurement d'un traitement à jets d'eau haute pression sur au moins un côté de la nappe de fibres afin d'entremêler la fibre de pâte à papier et la fibre chimique l'une avec l'autre, une teneur en fibre de pâte à papier de la première couche de fibres allant de 30 % en masse à 70 % en masse, une teneur en fibre chimique de la première couche de fibres étant de 70 % en masse et de 30 % en masse, une longueur de fibre moyenne de la fibre chimique ne faisant pas plus de 20 mm ;

à former une deuxième couche de fibres à partir de fibre de pâte à papier et de fibre chimique, la deuxième couche de fibres incluant de la fibre de pâte à papier, une teneur en fibre de pâte à papier de la deuxième couche de fibres allant de 70 % en masse à 100 % en masse ;

**caractérisée par** l'étape consistant à inclure dans la deuxième couche de fibres de la fibre chimique ne faisant pas plus de 30 % en masse avec une longueur de fibre moyenne de 20 mm ou moins et avec un diamètre de fibre plus petit que celui de la fibre de pâte à papier ; et

à transporter la première couche de fibres, à stratifier ultérieurement de façon solidaire la première couche de fibres et la deuxième couche de fibres l'une sur l'autre tandis que la deuxième couche de fibres demeure humide, et à sécher par la suite les première et deuxième couches de fibres stratifiées.

12. La méthode de fabrication d'une feuille d'emballage selon la revendication 11, comprenant en sus les étapes consistant :

à former une troisième couche de fibres à partir de fibre de pâte à papier et de fibre chimique en une nappe de fibres selon une méthode de formation de papier humide, et par application ultérieurement d'un traitement à jets d'eau haute pression sur au moins un côté de la nappe de fibres afin d'entremêler la fibre de pâte à papier et la fibre chimique l'une avec l'autre ; et

à transporter les première et deuxième couches de fibres après avoir stratifié de façon solidaire la première couche de fibres et la deuxième couche de fibres l'une sur l'autre tandis que les première et deuxième couches de fibres demeurent humides, à stratifier ultérieurement de façon solidaire la troisième couche de fibres sur les première et deuxième couches de fibres tandis que la troisième couche de fibres demeure humide, et à sécher par la suite les première à troisième couches de fibres stratifiées.

13. La méthode de fabrication d'une feuille d'emballage selon l'une quelconque des revendications 11 et 12, où dans l'une au moins des étape consistant à former la première couche de fibres et étape consistant à former la troisième couche de fibres, la nappe de fibres est transférée d'une toile de formation de couche de fibres à une toile de formation de motif une fois la nappe de fibres formée, et ultérieurement le traitement à jets d'eau haute pression est appliqué sur la nappe de fibres ainsi transférée.

14. La méthode de fabrication d'une feuille d'emballage selon l'une quelconque des revendications 11 à 13, où, pour l'une au moins des première et troisième couches de fibres, la nappe de fibres est formée par une méthode de formation de papier de n'importe quel type parmi un type tanmo et un type fourdrinier.

15. La méthode de fabrication d'une feuille d'emballage selon l'une quelconque des revendications 11, 12 et 14, où le traitement à jets d'eau haute pression est appliqué sur la nappe de fibres placée sur la toile de formation de couche de fibres.

16. La méthode de fabrication d'une feuille d'emballage selon l'une quelconque des revendications 11 à 15, où, pour la deuxième couche de fibres, la nappe de fibres est formée par une méthode de formation de papier de n'importe quel type parmi un type à cylindre et un type à formeur.

**FIG.1**

**FIG.2**

**FIG.3**

1

20
26

10

10a   13   29   27   10b

Y1

**FIG.4**

1   29

28

(a)

20   10   11   X ——— X'

26

10a   14   10b

X   24   25

(b)

22
21

X'   24   25

**FIG.5**

**FIG.6**

(a)

(b)

**FIG.7**

**FIG.8**

**EP 2 163 378 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI9228214 B
- JP 2001172850 A
- JP 9228214 A
- JP 2000318076 A